# EUROPEAN PATENT SPECIFICATION

(11) **EP 1 450 611 B1**
(45) Date of publication and mention of the grant of the patent: **06.07.2011**
(21) Application number: 02794219.2
(22) Date of filing: 10.12.2002
(51) Int. Cl.: A01N 63/00, A61K 48/00, C12N 15/00, C12N 15/09

(54) **METHOD FOR THE GENERATION OF ANTIGEN-SPECIFIC LYMPHOCYTES**
VERFAHREN ZUR HERSTELLUNG VON ANTIGENSPEZIFISCHEN LYMPHOZYTEN
PRODUCTION DE LYMPHOCYTES SPECIFIQUES D'UN ANTIGENE

(30) Priority: 10.12.2001 US 339375 P; 08.07.2002 US 394803 P
(43) Date of publication of application: 01.09.2004
(73) Proprietor: CALIFORNIA INSTITUTE OF TECHNOLOGY, Pasadena, CA 91125 (US)
(72) Inventor: YANG, Lili, Pasadena, CA 91106 (US); VAN PARIJS, Luk, Pasadena, CA 91125 (US); BALTIMORE, David, Pasadena, CA 91106 (US)
(74) Representative: Becker Kurig Straus
(86) International application number: PCT/US2002/039527
(87) International publication number: WO 2003/050262

(56) References cited:
- US-A- 5 830 755
- KESSELS H W ET AL: "Immunotherapy through TCR gene transfer." NATURE IMMUNOLOGY. OCT 2001, vol. 2, no. 10, October 2001 (2001-10), pages 957-961, XP002349011 ISSN: 1529-2908
- HOZUMI K ET AL: "Establishment of efficient reaggregation culture system for gene transfection into immature T cells by retroviral vectors." IMMUNOLOGY LETTERS. 10 JAN 2000, vol. 71, no. 1, 10 January 2000 (2000-01-10), pages 61-66, XP002349012 ISSN: 0165-2478
- CLAY T M ET AL: "Efficient transfer of a tumor antigen-reactive TCR to human peripheral blood lymphocytes confers anti-tumor reactivity." JOURNAL OF IMMUNOLOGY (BALTIMORE, MD. : 1950) 1 JUL 1999, vol. 163, no. 1, 1 July 1999 (1999-07-01), pages 507-513, XP002169636 ISSN: 0022-1767
- STANISLAWSKI T ET AL: "Circumventing tolerance to a human MDM2-derived tumor antigen by TCR gene transfer." NATURE IMMUNOLOGY. OCT 2001, vol. 2, no. 10, October 2001 (2001-10), pages 962-970, XP001086673 ISSN: 1529-2908
- YANG LILI ET AL: "Generation of functional antigen-specific T cells in defined genetic backgrounds by retrovirus-mediated expression of TCR cDNAs in hematopoietic precursor cells." PROCEEDINGS OF THE NATIONAL ACADEMY OF SCIENCES OF THE UNITED STATES OF AMERICA. 30 APR 2002, vol. 99, no. 9, 30 April 2002 (2002-04-30), pages 6204-6209, XP002349013 ISSN: 0027-8424
- ROBBINS ET AL.: 'Viral vectors for gene therapy' PHARMACOL. THER. vol. 80, no. 1, 1998, pages 35 - 47, XP002927113
- POGULIS R.J. ET AL: 'Retroviral-mediated expression of an MHC class I-restricted T cell receptor in the CD8 T cell compartment of bone marrow-reconstituted mice.' HUMAN GENE THERAPY vol. 9, 1998, pages 2285 - 2297
- CLAY T.M. ET AL: 'Potential use of T cell receptor genes to modify hematopoietic stem cells for the gene therapy of cancer' PATHOLOGY ONCOLOGY RESEARCH vol. 5, 1999, pages 3 - 15
- COOPER L.J. ET AL: 'Transfer of specificity for human immunodeficiency virus type 1 into primary human T lymphocytes by introduction of T-cell receptor genes.' JOURNAL OF VIROLOGY vol. 74, 2000, pages 8207 - 8212
- MHASHILKAR A.M. ET AL: 'INHIBITION OF HUMAN IMMUNODEFICIENCY VIRUS TYPE 1 REPLICATION IN VITRO IN ACUTELY AND PERSISTENTLY INFECTED HUMAN CD4+ MONONUCLEAR CELLS EXPRESSING MURINE AND HUMANIZED ANTI-HUMAN IMMUNODEFICIENCY VIRUS TYPE 1 TAT SINGLE-CHAINVARIABLE FRAGMENT INTRABODIES' HUMAN GENE THERAPY vol. 10, 1999, pages 1453 - 1467
- FINNEY H.M. ET AL: 'Chimeric receptors providing both primary and costimulatory signaling in T cells from a single gene product' JOURNAL OF IMMUNOLOGY vol. 161, 1998, pages 2791 - 2797
- PANDYA S. ET AL: 'Lentivirus and foamy virus vectors: novel gene therapy tools' EXPERT OPINION ON BIOLOGICAL THERAPY vol. 1, 2001, pages 17 - 40
- ROBBINS PAUL B. ET AL: 'Consistent, persistent expression from modified retroviral vectors in murine hematopoietic stem cells' PROCEEDINGS OF THE NATIONAL ACADEMY OF SCIENCES OF THE UNITED STATES OF AMERICA vol. 95, August 1998, pages 10182 - 10187
- MAY CHAD ET AL: 'Therapeutic haemoglobin synthesis in beta-thalassaemic mice expressing lentivirus-encoded human beta-globin' NATURE vol. 406, 06 July 2000, pages 82 - 86

## Description

### Background of the Invention

### Reference to Related Applications

The present application claims priority under 35 U.S.C. §119(e) to U.S. Provisional Application No. 60/394,803, filed July 8, 2002 and U.S. Provisional Application No. 60/339,375, filed December 10, 2001.

### Field of the Invention

The invention relates generally to the fields of gene delivery and immunology, and more particularly to the delivery of genetic material to cells of the immune system.

### Description of the Related Art

The adaptive immune system of vertebrates defends the host against infection. T cells play the role of central organizer of the immune response by recognizing antigens through T cell receptors (TCR). The specificity of a T cell depends on the sequence of its T cell receptor. The genetic template for this receptor is created during T cell development in the thymus by the V(D)J DNA rearrangement process, which imparts a unique antigen specificity upon each TCR. The TCR plays an essential role in T cell function, development and survival. Genetic lesions that interfere with the generation of antigen receptors block T cell development and result in immunodeficiencies. Because of the importance of T cells in organizing the immune response, it is desirable to be able to generate T cells having a particular antigen specificity.

Currently, the only available method for the generation of an animal having a T cell with a defined antigen specificity is to introduce the gene encoding the desired T cell receptor into an embryo by pronuclear injection. This technique requires handling a large fragment of genomic DNA encoding the rearranged α and β and chains of the TCR, a significant amount of time, and can only be practiced in limited genetic backgrounds. Moreover, such a technique is not suitable for therapeutic applications.

The introduction of a TCR into peripheral blood cells has been reported recently (P.A. Moss (2001) Nature Immunology 2, 900-901; Kessels et al. (2001) Nature Immunology 2, 957-961 and Stanislawski et al. (2001) Nature Immunology 2, 962-970). In these studies, TCRá and TCRâ genes were introduced and stably expressed in mature T cells that had been activated with a mitogen and then infected with a retroviral vector. Using this approach, T cells derived from non-specific, heterogeneous populations were converted into T cells capable of responding to protein antigens and tumor tissues. However, these methods do not produce lymphocytes having a well-defined antigen-specificity. Importantly, the T cells that are engineered to express the TCRs are activated mature cells that already express an endogenous TCR of unknown specificity. Thus the introduction of transgenic TCRá and â chains will lead to the heterologous combinations with the endogenous chains. These heterologous TCRs will have unpredictable specificity and may produce autoimmune damage. Furthermore, the effector function of the engineered cells is defined by the conditions under which these cells are activated *in vitro,* which will limit the type of immune responses they can induce. In addition, only a fraction of activated T cells have the capacity to persist *in vivo* an extended period of time.

Berg et al., 1988 reported production of a TCRβ transgenic mouse and Bluthman et al., 1988 reported a whole TCR transgenic mouse. The generation of TCR transgenic animals has also been reported by Uematsu et al. (1988), Pircher et al. (1989), Mamalaki et al. (1993), Kouskoff et al. (1995), and Barnden et al. (1998).

A number of reports also address the need in the art for methods that can be used to generate T cells having a defined specificity, including: Dembic et al., 1986; Clay et al., 1999; Fujio et al., Immunol 2000 Jul 1; Kessels et al., Immunol 2001 Oct; Stanislawski et al., Immunol 2001 Oct; Cooper et al., Virol., 2000; and Moss, Immunol 2001 Oct.

Recently, adoptive T cell therapy using antigen-specific T cell clones has been used successfully for the treatment of cancer (Dudley et al. Science 298:850-854 (2002); Yee et al. Proc. Natl. Acad. Sci. USA, Early Edition 10.1073/pnas.242600099 (2002)).

Clay T.M. et al. ("Potential use of T cell receptor genes to modify hematopoietic stem cells fort he gene therapy of cancer", Pathology Oncology Research, Vol. 5, 1999, p. 3-15) disclose a method of introducing a T cell receptor (TCR) into a hematopoietic stem cell using a retroviral vector with a two-promoter system. The authors report on detecting the presence of DNA and RNA of an introduced exogenous TCR gene.

Kessels H.W. et al. ("Immunotherapy through TCR gene transfer", Nature Immunology, Vol. 2, 2001, p. 957-961) disclose introducing a virus-specific T cell receptor (TCR) into a mature peripheral T cell, i.e. into a splenocyte. T cells produced from the method disclosed in Kessels et al. have two TCRs: an endogenous TCR as well as a transgenic TCR.

Because of the importance of antigen specific T cells to the immune response and their usefulness in treating disease, there is a great need for techniques that enable the production of transgenic cells that have a defined antigen specificity. This invention addresses this and other needs in the art.

### Summary of the Invention

The invention provides methods for the generation of lymphocytes having unique antigen specificity. Lymphocytes generated according to the methods of the invention have a number of utilities, including therapeutic applications, such as priming an organism's immune response against a pathogen, and providing an immune response against a particular disease or disorder, such as diseased tissue, for example, cancerous tissue.

According to the preferred embodiment of the invention an in vitro method of generating a lymphocyte with a unique antigen specificity in a mammal is provided. The method comprises the step of contacting a mammalian stem cell in vitro with a modified lentivirus polynucleotide delivery system comprising an antigen-specific polynucleotide, encoding a T cell receptor α subunit and a T cell receptor β subunit under the control of one promoter. The mammalian stem cell to be used in the method is a hematopoietic stem cell, a primary bone marrow cell or a hematopoietic precursor cell.

According to a further embodiment of the invention a lymphocyte with a unique antigen specificity is provided which is obtainable by the method according to the invention.

In a further embodiment the present invention provides the use of a lymphocyte with a unique antigen specificity in the preparation of a medicament for stimulating an immune response to the antigen in a mammal.

In another embodiment of the invention a lymphocyte with a unique antigen specificity is provided which is obtainable by the method according to the invention, which method comprising the additional steps of cloning a T cell that expresses the T cell receptor on its surface obtained from a patient and expanding the T cell in vitro.

In yet another embodiment of the invention the lymphocyte with a unique antigen specificity is used in the preparation of a medicament for the treatment of cancer, wherein the unique antigen is associated with cancer.

In a further aspect the invention provides the use of a lymphocyte with a unique antigen specificity which is obtainable by the method according to the invention in the preparation of a medicament for preventing infection in a mammal that has been or is expected to be exposed to an infectious agent, wherein the antigen is associated with the infectious agent. Preferably the infectious agent is HIV.

### Brief Description of the Drawings

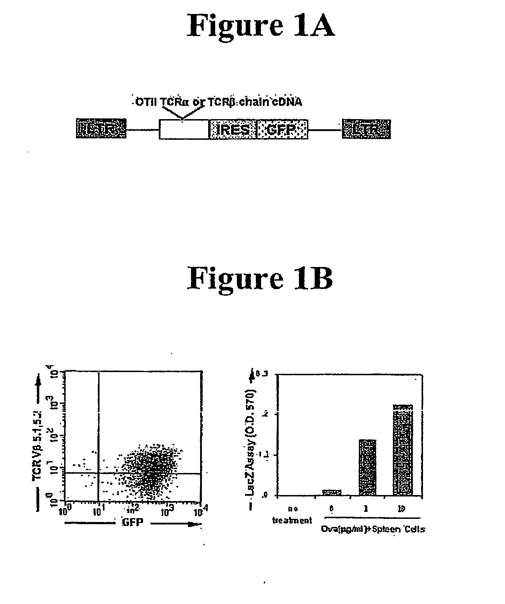

Figure 1A schematically illustrates a retroviral vector, MIG (MSCV IRES GFP), used as a polynucleotide delivery system. The illustrated vector expresses the cDNA for the OTII TCRá or TCRâ chain. The long terminal repeat (LTR), internal ribosomal entry site (IRES) and green fluorescent protein (GFP) regions of the vector are indicated.
Figure 1B illustrates surface expression of the OTII TCRâ chain in infected (GFP+) THZ cells and primary CD4+ cells. Cells were co-infected with MIG retroviruses expressing the cDNA for the OTII TCR α or β chain and then stained with a PE-conjugated antibody against TCR Vβ 5.1,5.2, which is the Vβ element used by the OTII TCRβ chain. Functional expression of the OTII TCR in THZ cells and primary CD4+ cells is also shown (right panel). Cells were co-infected with MIG retroviruses expressing OTII TCRα chain or OTII TCRβ chain and restimulated for 48 hours with OVAp in the presence of B6 spleen cells as APCs. Antigen response of THZ cells was assessed by assaying for the induction of â-galactosidase expression and by ³H-thymidine incorporation for primary CD4+ cells.

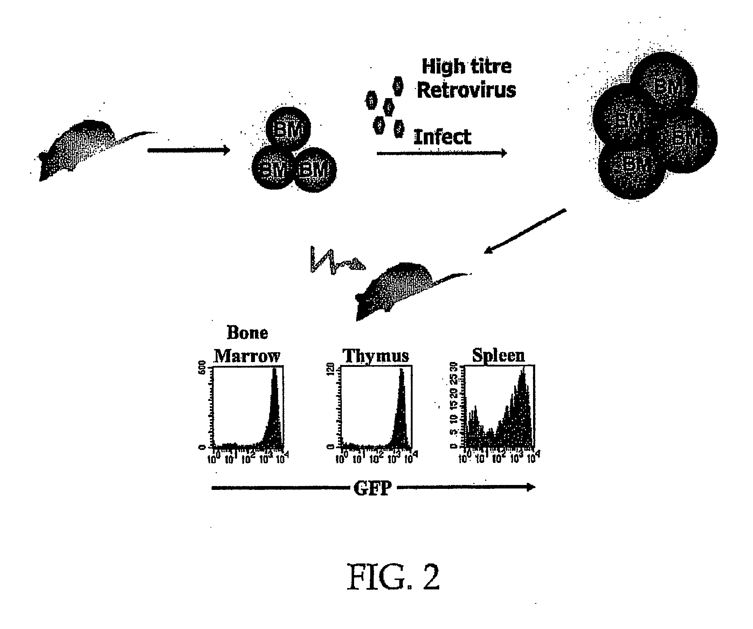

Figure 2 shows a diagram of the strategy to generate TCR transgenic T cells using retrovirus-based gene delivery into BM stem cells. Hematopoietic precursor cells were obtained from wild type and IL-2 deficient RAG knockout mice that had been treated with 5-fluorouracil. These cells were then cultured in the presence of cytokines and co-infected with MIG retroviruses expressing the cDNA for the OTII TCRα or β chain. The infected hematopoietic precursor cells were then transferred into a lethally irradiated host mouse and allowed to reconstitute the immune system. Cells expressing the retrovirally-encoded genes were identified by their expression of the green fluorescent protein.

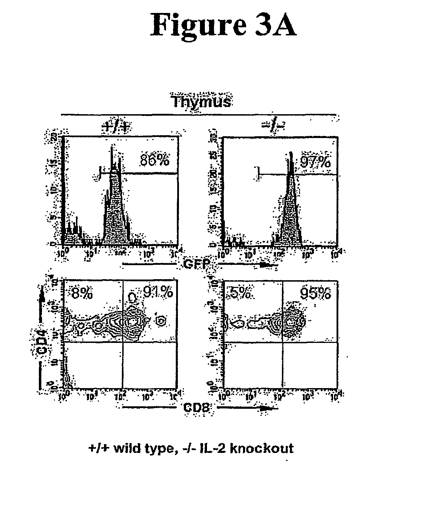

Figure 3A shows the normal development of OTII TCR transgenic CD4+ T cells in the thymus of mice receiving retrovirally-transduced bone marrow stem cells. Thymocytes obtained from lethally-irradiated host mice 11 weeks after injection of retrovirally-transduced hematopoietic precursor cell were stained with anti-CD4-Cyc and anti-CD8-PE antibodies and analyzed by flow cytometry. The distribution of CD4 and CD8 expression on GFP+ thymocytes is shown.

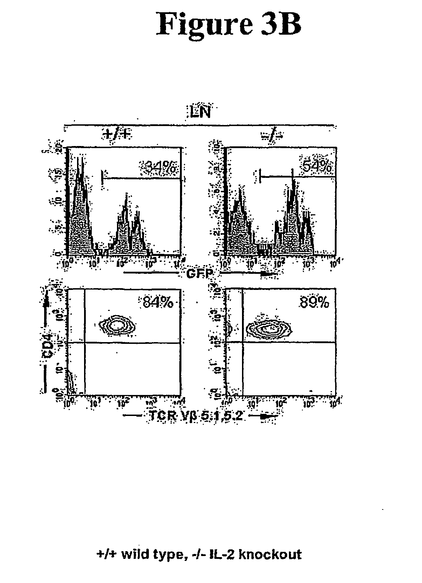

Figure 3B shows the presence of mature OTII TCR transgenic CD4+ T cells in the peripheral lymphoid organs of mice receiving retrovirally-transduced bone marrow stem cells. Lymph node and spleen (not shown) cells obtained from lethally irradiated host mice 11 weeks after injection of retrovirally-transduced hematopoietic precursor cells were stained with anti-CD4-Cyc and anti-TCR Vβ 5.1,5.2-PE antibodies and analyzed by flow cytometry. The distribution of CD4 and Vβ 5.1,5.2 expression on GFP+ lymph node cells is shown.

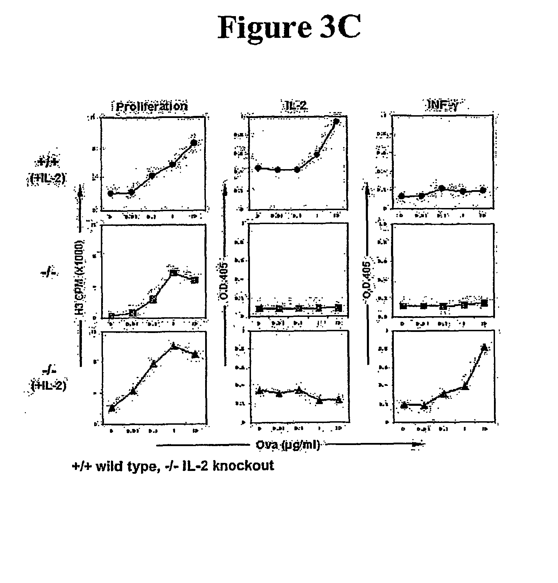

Figure 3C shows normal functional responses of OTII TCR transgenic CD4+ T cells obtained from the peripheral lymphoid organs of mice receiving retrovirally-transduced bone marrow stem cells. Spleen cells obtained from lethally irradiated host mice 11 weeks after injection of retrovirally-transduced hematopoietic precursor cells derived from IL-2 deficient mice were supplemented with B6 spleen cells as APCs and stimulated in vitro with OVAp in the presence or absence of exogenous IL-2. Proliferation was assayed after 72 hours by ³H-thymidine incorporation and cytokine production by ELISA. Data was normalized for the number of GFP+CD4+TCR Vβ 5.1,5.2+ cells present in the starting spleen cell populations. Proliferation and cytokine production was seen with wild type OTII T cells both in the presence and absence of IL-2 (data not shown).

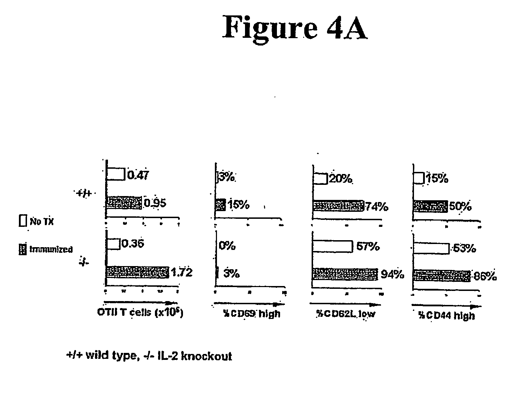

Figure 4A shows the normal cell expansion and expression of activation following *in vivo* antigen stimulation of OTII TCR transgenic CD4+ T cells in the peripheral lymphoid organs of mice receiving retrovirally-transduced bone marrow stem cells. Lethally-irradiated host mice were immunized via an intra peritoneal injection of 200 i g OVAp or left untreated (No TX) 10 weeks after receiving retrovirally-transduced hematopoietic precursor cells. Spleen and lymph node cells were harvested and counted 6 days later. An aliquot of these cells was stained with anti-CD4-Cyc and anti-TCR Vβ 5.1,5.2-PE, anti-CD62L-PE or anti-CD44-PE antibodies and analyzed by flow cytometry. The number of OTII TCR transgenic T cells present in the spleen and lymph nodes of immunized and control mice was determined by multiplying the percentage of GFP+CD4+TCR Vβ 5.1,5.2+ cells by the total number of cells present in these organs. The frequency of activated T cells was determined by gating on GFP+ CD4+TCR Vβ 5.1,5.2_ and CD62L low or CD44 high cells.

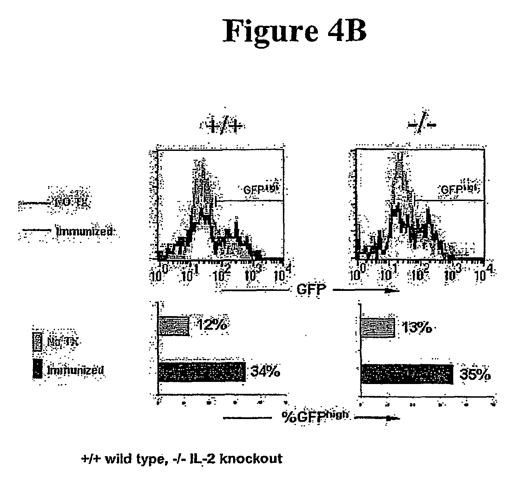

Figure 4B shows the preferential expansion of GFP^{high} OTII TCR transgenic CD4+ T cells following stimulation with antigen *in vivo.* Mice receiving retrovirally-transduced hematopoietic precursor cells were immunized as in (A). Spleen and lymph node cells were collected and stained with anti-CD4-Cyc and anti-TCR Vβ 5.1,5.2-PE antibody and analyzed by flow cytometry. The expression of GFP in Vβ 5.1,5.2_ CD4+ OTII T cells, and the frequency of GFP^{high} OTII T cells is shown.

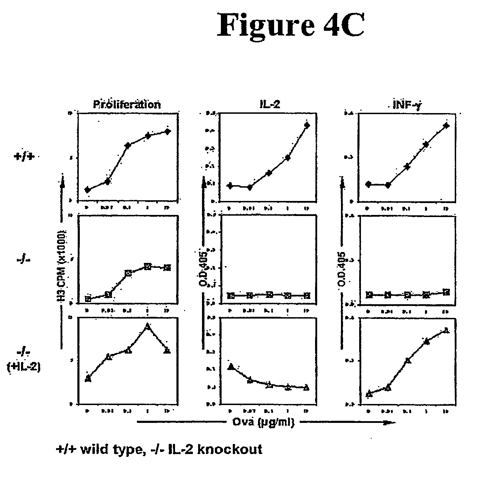

Figure 4C shows normal functional responses of OTII TCR transgenic CD4+ T cells following *in vivo* stimulation with antigen. Mice receiving retrovirally-transduced hematopoietic precursor cells were immunized as in (A). Spleen/LN cells were harvested and stimulated *in vitro* with OVAp in the presence of B6 spleen cells as APCs. Proliferation was assayed by ³H-thymidine incorporation, cytokines by ELISA. Data was normalized for the number of GFP+ CD4+ TCR Vβ 5.1,5.2+ cells present in the starting spleen cell populations.

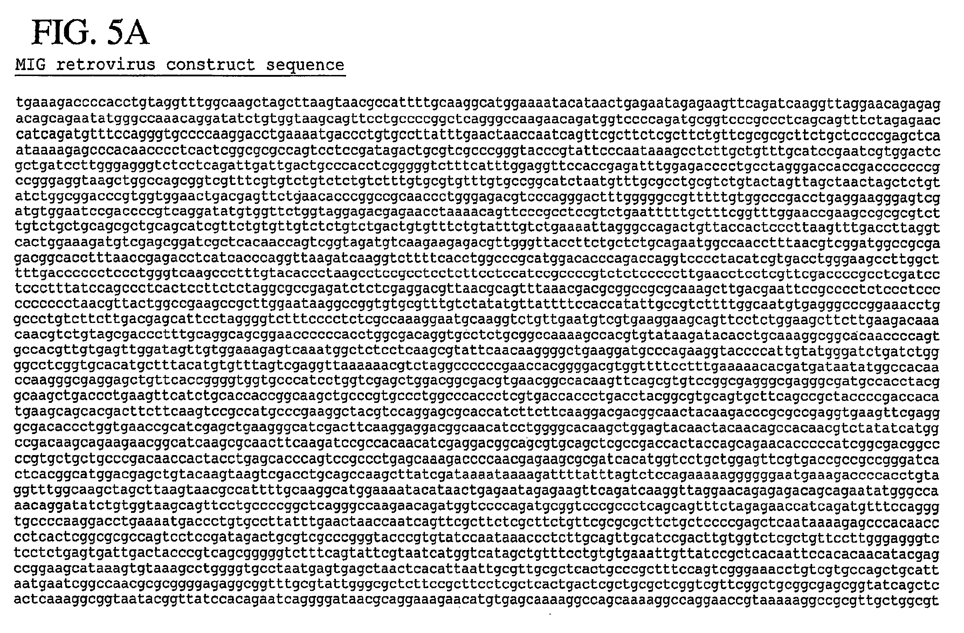

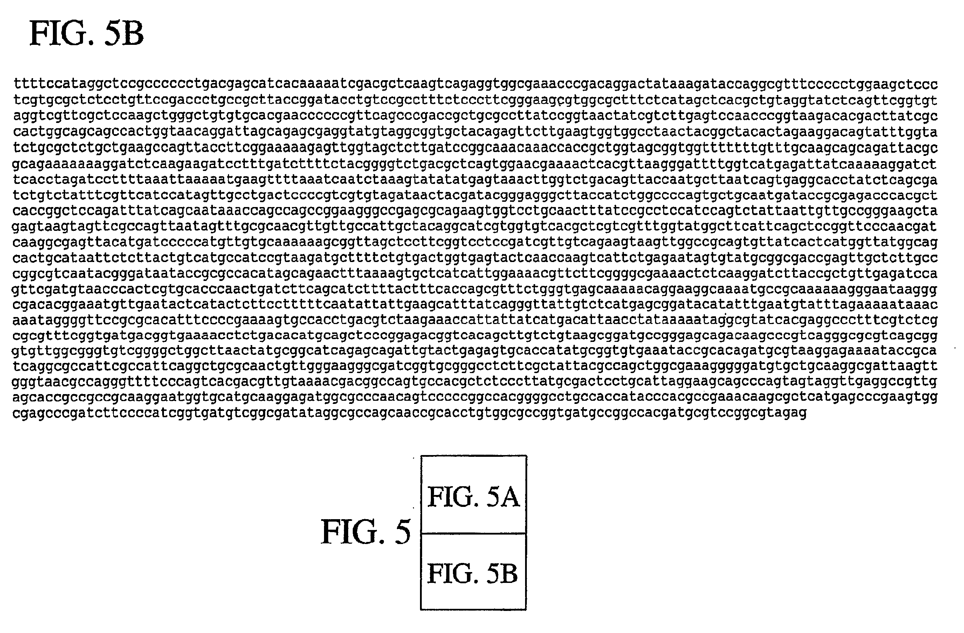

Figures 5A and 5B provide the sequence of the MIG retrovirus construct (SEQ ID NO: 1).

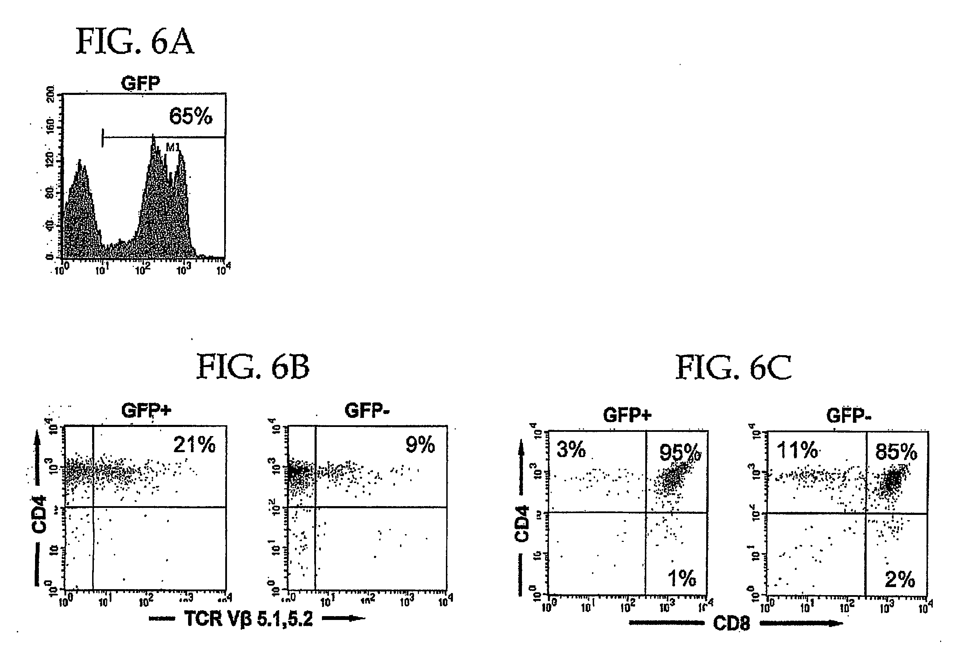

Figures 6A-C show that retrovirus mediated transfer into bone marrow from wild type mice generates thymocytes expressing transgenic OTII TCR. Cells were obtained from the thymus of mice that received wild type bone marrow infected with recombinant retrovirus. Cells were analyzed for expression of GFP, TCR β, CD4 and CD8.

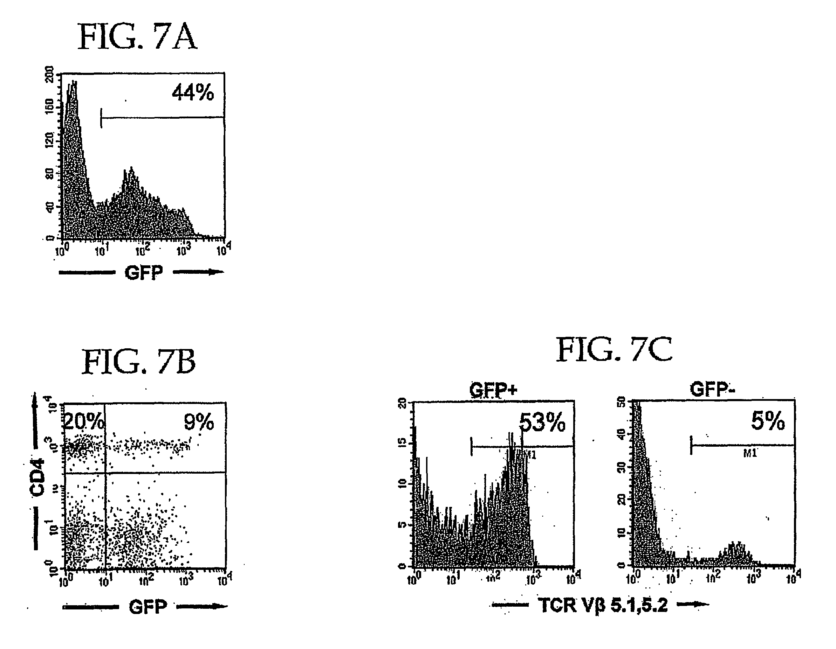

Figures 7A-C show that retrovirus mediated transfer into bone marrow from wild type mice generates mature CD4+ T cells that express transgenic TCR in the periphery. Cells were obtained from the peripheral lymph nodes of mice receiving wild type bone marrow that had been infected with recombinant retrovirus. Cells were analyzed for GFP, CD4 and TCRβ expression.

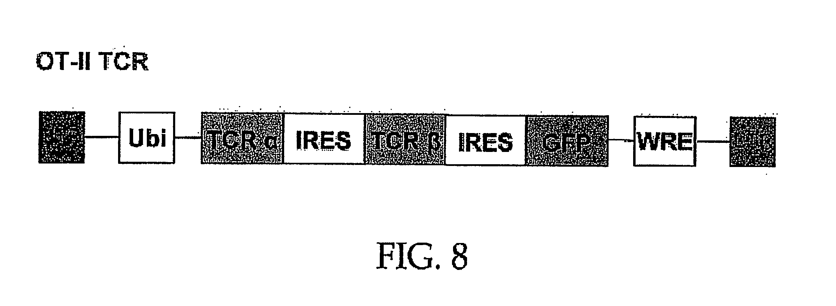

Figure 8 is a diagram of a lentiviral construct that is used to produce recombinant lentivirus. The tri-cistronic construct comprises sequence encoding the OTII TCR α and β chains, as well as a GFP marker gene. The genes are separated by an internal ribosome entry site (IRES) sequence. Recombinant virus is produced in a packaging cell line and used to infect cells in which T cell receptor expression is desired.

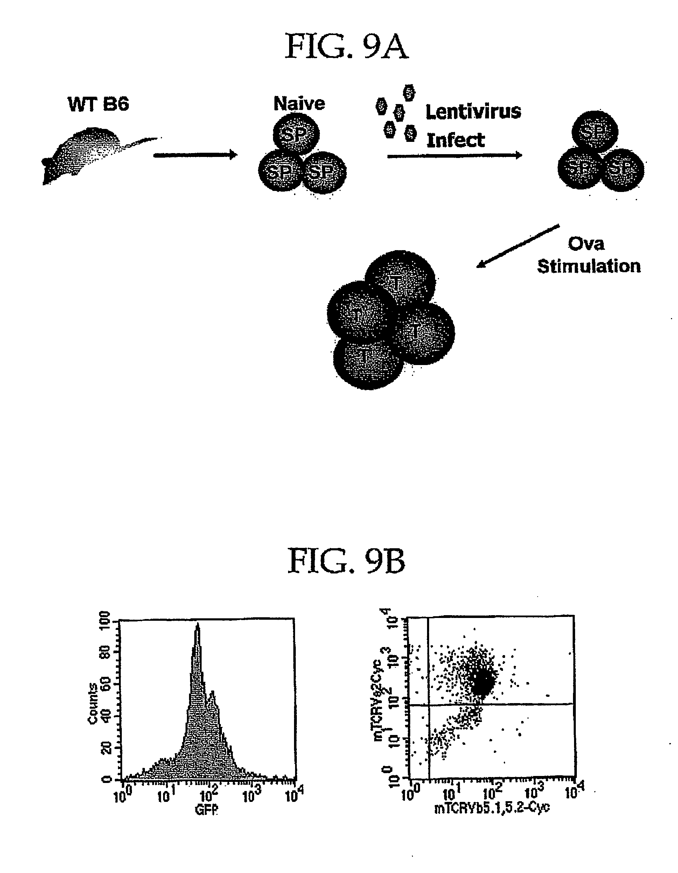

Figure 9A diagrams the method of infection of naive T cells with the tri-cistronic lentivirus comprising OTII TCRα, β and GFP. Naive spleen cells are obtained from wild type B6 mice and infected with recombinant lentivirus. The cells are then stimulated with ova and their response is measured. As can be seen in Figure 9B, nearly all cells are GFP positive and greater than 90% express OTII TCRα and β and respond to antigen stimulation.

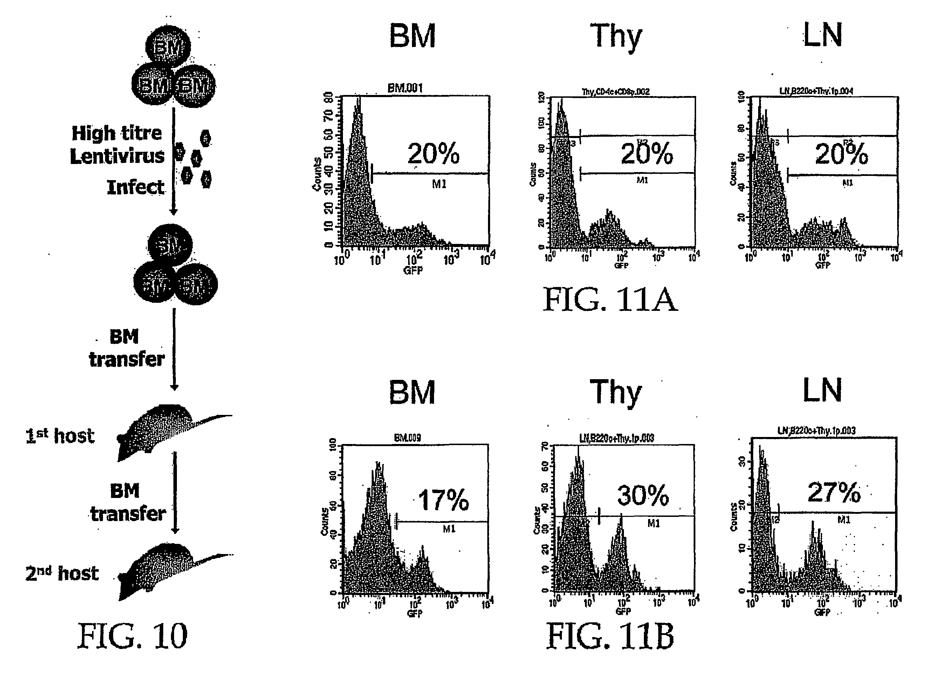

Figure 10 diagrams the method of producing modified T cells in wild type animals Wild type bone marrow cells are infected with lentivirus comprising the OTII TCRα and β chain and the GFP marker. The bone marrow is transferred into a wild type, non-irradiated mouse, the first host. Bone marrow from the first house is transferred into a second wild type mouse, the second host. Cells from the first and second host are analyzed for expression of the GFP marker gene.
Figures 11A and B show that cells from the bone marrow (BM), thymus (Thy) and peripheral lymph nodes (LN) of both the first (Fig. 11A) and second (Fig. 11B) host treated as in Figure 10, express the GFP transgene, indicating that the gene is stably integrated in the hematopoietic stem cells.

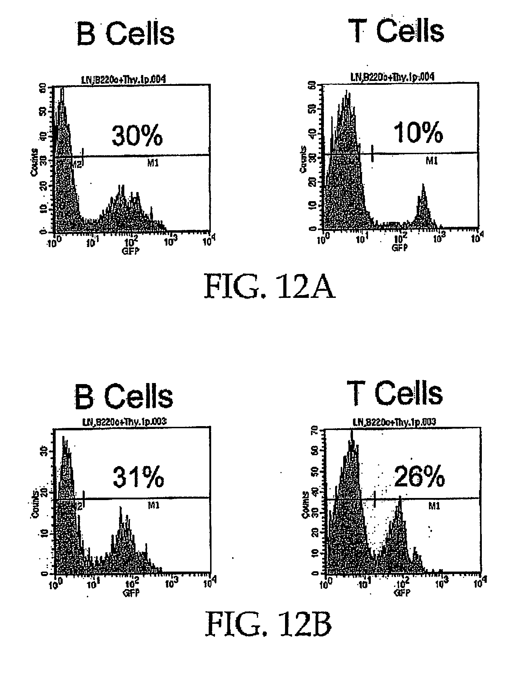

Figures 12A and B show that lentiviral infection of fresh bone marrow (BM) mediated stable gene transfer into hematopoietic stem cells. Approximately 30% of B cells, from the first host (Fig. 12A) and 10% of T cells express GFP, while approximately 31 % of B cells and 26% of T cells from the second host express GFP (Fig. 12B).

### Detailed Description of the Preferred Embodiment

The present invention is based on the experimental finding that it is possible to obtain functional T cells with a desired antigen specificity by expression of TCRα and β cDNAs in hematopoietic stem cells.

Methods are provided for generating immune cells with desired antigen specificity. According to one aspect of the invention, immune cells with antigen specificity are generated by transfecting an appropriate target cell with an antigen-specific polynucleotide.

In a preferred embodiment of the invention a lymphocyte with a unique antigen specificity in a mammal is generated. This method comprises the step of contacting a mammalian stem cell in vitro with a modified lentivirus polynucleotide delivery system comprising an antigen-specific polynucleotide, encoding a T cell receptor α subunit and a T cell receptor β subunit under the control of one promoter. The mammalian stem cell to be used is a hematopoietic stem cell, a primary bone marrow cell or a hematopoietic precursor cell.

### A. Definitions

Unless defined otherwise, technical and scientific terms used herein have the same meaning as commonly understood by one of ordinary skill in the art to which this invention belongs. See, e.g. Singleton et al., Dictionary of Microbiology and Molecular Biology 2nd ed., J. Wiley & Sons (New York, NY 1994); Sambrook et al., Molecular Cloning A Laboratory Manual, Cold Springs Harbor Press (Cold Springs Harbor, NY 1989). Any methods, devices and materials similar or equivalent to those described herein can be used in the practice of this invention.

As used herein, the terms nucleic acid, polynucleotide and nucleotide are interchangeable and refer to any nucleic acid, whether composed of phosphodiester linkages or modified linkages such as phosphotriester, phosphoramidate, siloxane, carbonate, carboxymethylester, acetamidate, carbamate, thioether, bridged phosphoramidate, bridged methylene phosphonate, bridged phosphoramidate, bridged phosphoramidate, bridged methylene phosphonate, phosphorothioate, methylphosphonate, phosphorodithioate, bridged phosphorothioate or sultone linkages, and combinations of such linkages.

The terms nucleic acid, polynucleotide and nucleotide also specifically include nucleic acids composed of bases other than the five biologically occurring bases (adenine, guanine, thymine, cytosine and uracil).

As used herein, a nucleic acid molecule is said to be "isolated" when the nucleic acid molecule is substantially separated from contaminant nucleic acid molecules encoding other polypeptides.

An "antigen" is any molecule that is capable of binding to an antigen specific polypeptide. Preferred antigens are capable of initiating an immune response upon binding to an antigen specific polypeptide that is expressed in an immune cell. An "immune response" is any biological activity that is attributable to the binding of an antigen to an antigen specific polypeptide.

The term "epitope" is used to refer to a site on an antigen that is recognized by an antigen specific polypeptide.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules that lack antigen specificity. Polypeptides of the latter kind are, for example, produced at low levels by the lymph system and at increased levels by myelomas.

"Native antibodies" and "native immunoglobulins" are usually heterotetrameric glycoproteins, composed of two identical light (L) chains and two identical heavy (H) chains. Each light chain is linked to a heavy chain by a disulfide bond. The number of disulfide linkages varies among the heavy chains of different immunoglobulin isotypes. Each heavy chain comprises a variable domain (V_{H)} followed by a number of constant domains. Each light chain comprises a variable domain at one end (V_{L}) and a constant domain at its other end. The constant domain of the light chain is aligned with the first constant domain of the heavy chain, and the light- chain variable domain is aligned with the variable domain of the heavy chain.

The term "antibody" herein is used in the broadest sense and specifically covers human, non-human (e.g. murine) and humanized monoclonal antibodies (including full length monoclonal antibodies), polyclonal antibodies, multi-specific antibodies (*e.g*., bispecific antibodies), and antibody fragments so long as they exhibit the desired biological activity.

T cell receptors ("TCRs") are complexes of several polypeptides that are able to bind antigen when expressed on the surface of a cell, such as a T lymphocyte. The α and β chains, or subunits, form a dimer that is independently capable of antigen binding. The α and β subunits typically comprise a constant domain and a variable domain.

As used herein, the term "T cell receptor" includes a complex of polypeptides comprising a T cell receptor α subunit and a T cell receptor β subunit. The α and β subunits may be native, full-length polypeptides, or may be modified in some way, provided that the T cell receptor retains the ability to bind antigen. For example, the α and β subunits may be amino acid sequence variants, including substitution, addition and deletion mutants. They may also be chimeric subunits that comprise, for example, the variable regions from one organism and the constant regions from a different organism.

"Target cells" are any cells that are capable of expressing an antigen-specific polypeptide on their surface. Preferably, target cells are capable of maturing into immune cells, such as lymphocytes. Target cells include stem cells, particularly hematopoietic stem cells.

As used herein, a cell exhibits a "unique antigen specificity" if it is primarily responsive to a single type of antigen.

The term "mammal" is defined as an individual belonging to the class Mammalia and includes, without limitation, domestic and farm animals, and zoo, sports, or pet animals, such as sheep, dogs, horses, cats or cows.

A "subject" is any mammal that is in need of treatment.

As used herein, "treatment" is a clinical intervention made in response to a disease, disorder or physiological condition manifested by a patient or to be prevented in a patient. The aim of treatment includes the alleviation and/or prevention of symptoms, as well as slowing, stopping or reversing the progression of a disease, disorder, or condition. "Treatment" refers to both therapeutic treatment and prophylactic or preventative measures. Those in need of treatment include those already affected by a disease or disorder or undesired physiological condition as well as those in which the disease or disorder or undesired physiological condition is to be prevented.

"Tumor," as used herein, refers to all neoplastic cell growth and proliferation, whether malignant or benign, and all pre-cancerous and cancerous cells and tissues.

The term "cancer" refers to a disease or disorder that is characterized by unregulated cell growth. Examples of cancer include, but are not limited to, carcinoma, lymphoma, blastoma and sarcoma. Examples of specific cancers include, but are not limited to, lung cancer, colon cancer, breast cancer, testicular cancer, stomach cancer, pancreatic cancer, ovarian cancer, liver cancer, bladder cancer, colorectal cancer, and prostate cancer. Additional cancers are well known to those of skill in the art.

A "vector" is a nucleic acid molecule that is capable of transporting another nucleic acid. Vectors may be, for example, plasmids, cosmids or phage. An "expression vector" is a vector that is capable of directing the expression of a protein encoded by one or more genes carried by the vector when it is present in the appropriate environment. Vectors are preferably capable of autonomous replication.

The term "regulatory element" and "expression control element" are used interchangeably and refer to nucleic acid molecules that can influence the expression of an operably linked coding sequence in a particular host organism. These terms are used broadly to and cover all elements that promote or regulate transcription, including promoters, core elements required for basic interaction of RNA polymerase and transcription factors, upstream elements, enhancers, and response elements (see, e.g., Lewin, "Genes V" (Oxford University Press, Oxford) pages 847-873). Exemplary regulatory elements in prokaryotes include promoters, operator sequences and a ribosome binding sites. Regulatory elements that are used in eukaryotic cells may include, without limitation, promoters, enhancers, splicing signals and polyadenylation signals.

The term "transfection" refers to the introduction of a nucleic acid into a host cell by nucleic acid-mediated gene transfer, such as by contacting the cell with a polynucleotide delivery system as described below. "Transformation" refers to a process in which a cell's genetic make up is changed by the incorporation of exogenous nucleic acid.

By "transgene" is meant any nucleotide or DNA sequence that is integrated into one or more chromosomes of a target cell by human intervention. In one embodiment the transgene comprises an antigen-specific polynucleotide that encodes an antigen-specific polypeptide whose expression in a target cell is desired. The antigen-specific polynucleotide is generally operatively linked to other sequences that are useful for obtaining the desired expression of the gene of interest, such as transcriptional regulatory sequences. In another embodiment the transgene can additionally comprise a DNA sequence that is used to mark the chromosome where it has integrated.

The term "transgenic" is used herein to describe the property of harboring a transgene. For instance, a "transgenic organism" is any animal, including mammals, fish, birds and amphibians, in which one or more of the cells of the animal contain nucleic acid introduced by way of human intervention. In the typical transgenic animal, the transgene causes the cell to express or overexpress a recombinant protein.

"Retroviruses" are enveloped RNA viruses that are capable of infecting animal cells. "Lentivirus" refers to a genus of retroviruses that are capable of infecting dividing and non-dividing cells. Several examples of lentiviruses include HIV (human immunodeficiency virus; including HIV type 1, and HIV type 2), visna-maedi, the caprine arthritis-encephalitis virus, equine infectious anemia virus, feline immunodeficiency virus (FIV), bovine immune deficiency virus (BIV), and simian immunodeficiency virus (SIV).

"Transformation," as defined herein, describes a process by which exogenous DNA enters a target cell. Transformation may rely on any known method for the insertion of foreign nucleic acid sequences into a prokaryotic or eukaryotic host cell. The method is selected based on the type of host cell being transformed and may include, but is not limited to, viral infection, electroporation, heat shock, lipofection, and particle bombardment. "Transformed" cells include stably transformed cells in which the inserted DNA is capable of replication either as an autonomously replicating plasmid or as part of the host chromosome. Also included are cells that transiently express the antigen specific polypeptide.

### Antigens

The methods and compositions of the invention can be used to develop an immune response within an organism that is directed against a particular antigen of interest, such as an antigen that is associated with a disease or disorder. Thus, an antigen is preferably identified that is associated with a disease or disorder of interest, such as a disease or disorder that is to be treated in a patient. Once an antigen has been identified, an antigen-specific polynucleotide is identified such that expression of the antigen-specific binding protein encoded by the antigen-specific polynucleotide will cause a cell to be targeted to the desired antigen.

The antigen is not limited in any way and is preferably chosen based on the desired immune response. Antigens may be, for example, polypeptides, carbohydrates, lipids or nucleic acids. Examples of antigens to which an immune response can be developed include, without limitation, tumor antigens, viral antigens, microbial antigens, allergens, and autoantigens. In one embodiment, the antigen is a viral antigen, such as an HIV antigen. In another embodiment the antigen is a tumor associated antigen (TAA).

In a preferred embodiment an immune response is to be generated against a tumor associated antigen, such as in a mammal that has a tumor or other cancer or disease that is associated with a tumor associated antigen. Tumor associated antigens are known for a variety of diseases including, for example, prostate cancer and breast cancer. In some breast cancers, for example, the Her-2 receptor is overexpressed on the surface of cancerous cells. A number of tumor associated antigens have been reviewed (see, for example, "Tumor-Antigens Recognized By T-Lymphocytes," Boon T, Cerottini JC, Vandeneynde B, Vanderbruggen P, Vanpel A, Annual Review Of Immunology 12: 337-365, 1994; "A listing of human tumor antigens recognized by T cells," Renkvist N, Castelli C, Robbins PF, Parmiani G. Cancer Immunology Immunotherapy 50: (1) 3-15 MAR 2001).

### Antigen-specific polypeptides and polynucleotides

Once an antigen of interest has been selected, an antigen-specific polypeptide that is capable of interacting with the antigen is preferably identified, along with the antigen-specific polynucleotide that encodes it. An "antigen-specific polypeptide" or "antigen-specific binding protein" is a polypeptide that is capable of selectively binding to a particular antigen. That is, it binds to one antigen but does not substantially bind to other antigens. The term "antigen-specific polypeptide" encompasses both single polypeptides and a number of independent polypeptides that interact, as in a multi-subunit receptor. A preferred "antigen specific polypeptide" is a T cell receptor, particularly a T cell receptor that comprises an α subunit and a β subunit. When expressed on the surface of a cell the antigen-specific polypeptide is capable of causing the cell to selectively interact with a desired antigen. If the cell is of the appropriate type, such as an immune cell, particularly a lymphocyte, the selective interaction may generate an immune response.

An "antigen-specific polynucleotide" is a polynucleotide that encodes an antigen-specific polypeptide. The antigen specific polynucleotide may encode more than one polypeptide. For example, the antigen specific polynucleotide may encode all of the subunits of a multi-subunit receptor.

An antigen-specific polynucleotide may comprise a single polynucleotide molecule. However, an "antigen-specific polynucleotide" may comprise more than one independent polynucleotide molecule, particularly when it encodes an antigen-specific polypeptide that comprises more that one subunit. In this case, each subunit may be encoded by a separate polynucleotide. All of the subunits may alternatively be encoded by a single polynucleotide.

An antigen-specific polynucleotide can be derived from any source, but is preferably derived from a genomic DNA sequence or a cDNA sequence of a gene. In addition, the antigen-specific polynucleotide can be produced synthetically or isolated from a natural source. Antigen-specific polynucleotides may comprise, without limitation, DNA, cDNA and/or RNA sequences that encode antigen-specific polypeptides. Preferably, the antigen-specific polynucleotides used in the methods of the present invention comprise cDNA sequences.

It is understood that all polynucleotides encoding a desired antigen-specific polypeptide are included herein. Such polynucleotides include, for example, naturally occurring, synthetic, and intentionally manipulated polynucleotides. For example, the antigen-specific polynucleotide may be a naturally occurring polynucleotide that has been subjected to site-directed mutagenesis. Also included are naturally occurring antigen-specific polynucleotides that comprise deletions, insertions or substitutions, so long as they encode antigen-specific polypeptides that retain the ability to interact with the antigen.

The antigen-specific polynucleotides also include sequences that are degenerate as a result of the genetic code. There are 20 natural amino acids, most of which are specified by more than one codon. Therefore, all degenerate nucleotide sequences are included in the invention as long as the encoded polypeptide has the desired specificity.

The polynucleotide sequence may be a cDNA sequence. The polynucleotide sequence can also be a cDNA sequence that has been intentionally manipulated, such as a cDNA that has been mutated to remove potential splice sites or to match codon usage to a particular host organism. Such manipulations are within the ordinary skill in the art.

The antigen-specific polynucleotide may encode an antigen specific polypeptide that is a cell surface receptor. The antigen specific polynucleotide can also encode one or more antigen-specific polypeptides selected from the group consisting of T cell receptors and immunoglobulins, including, without limitation, B cell receptors (BCR), single chain antibodies, and combinations thereof.

The polynucleotide sequence of an antigen specific polypeptide, such as a receptor that is specific for a given antigen, can be determined or generated by any technique known in the art. The antigen specific polypeptide may be a T cell receptor (TCR). One technique available for obtaining the polynucleotide sequence of a T cell receptor is to isolate T cells that bind to a specific antigen and to determine the sequence of the T cell receptor (TCR) encoded by that isolated clone. This method is well known in the art.

When a TCR sequence is determined in an organism other than that from which the target cells in which it is to be expressed are derived, it is possible to clone out the whole TCR. However, a preferred method is to clone out the sequence of the variable regions of the TCR subunits. Then the variable sequences are linked to the sequence of the TCR gene constant regions from the organism from which the target cells are derived to obtain an antigen-specific polynucleotide. The hybrid TCR expressed from this antigen-specific polynucleotide has the desired antigen specificity, but originates from the same organism as the target cells.

A TCR that recognizes an antigen of interest may also be identified. An antigen of interest, such as a protein or peptide, is identified, for example a tumor specific antigen (for one type of tumor or several types of tumor). The antigen is used to immunize a humanized mouse that express certain human HLA allele(s). T cell clones are generated that respond to the tumor antigen, which are restricted by the expressed human HLA allele(s). TCRs are then cloned from these T cell clones. A single antigen-specific polynucleotide encoding a TCR that recognizes the antigen of interest may be identified and transferred into target cells using a polynucleotide delivery system as described below. The target cells may then be transferred into a mammal in which an immune response to the antigen is desired.

Alternatively, a TCR library of polynucleotides encoding TCRs with desired properties (e.g. high antigen responsiveness and/or the ability to collaborate with each other) may be established from the T cell clones. The TCRs may be whole cloned TCRs or hybrid TCRs as described above. The TCR library may then be delivered into target cells, one TCR per fraction, to generate antigen-specific T cells. This can be accomplished, for example, using the techniques described for a single gene (not a library) by Stanislawski, 2001, "Circumventing tolerance to a human MDM2-derived tumor antigen by TCR gene transfer." Nature Immunol. 2,962-70.

When the antigen-specific polypeptide is not a TCR, other techniques can be used to identify an antigen-specific polynucleotide sequence. For example, when the antigen-specific polypeptide is an immunoglobulin, the antigen-specific polynucleotide sequence can be derived from the sequence of a monoclonal antibody that specifically binds the antigen. The antigen-specific antibody can comprise the entire antibody. However, if the antigen-specific polypeptide is to be used to generate an immune response in a mammal, the antibody sequence will preferably be fused to a membrane-spanning domain and appropriate signaling peptides. Alternatively, an antigen-specific polypeptide comprising an antibody fragment can be used, such as by grafting the antibody fragment to a membrane spanning region and appropriate signaling sequences.

The antigen-specific polypeptide may comprise the variable region responsible for the interaction of an antibody with an antigen. For example, the variable region may be grafted into the sequence of a B cell receptor sequence.

In these and similar ways, a monoclonal antibody from an organism other than that from which the target cells are derived can be used to generate an antigen-specific polypeptide that is specific to the target cell organism. Other techniques known in the art for generating diversity in a receptor can also be used.

Antigen-specific polynucleotides can also be generated by a variety of molecular evolution and screening techniques, including, for example, exon shuffling and phage display. For example, when the antigen-specific polypeptide is an immunoglobulin, including both single chain and dual chain antibodies, a polynucleotide encoding the immunoglobulin specific for a given antigen can be selected using phage display techniques. Phage display can be performed in a variety of formats; for their review see, *e.g.,* Johnson, Kevin S. and Chiswell, David J., Current Opinion in Structural Biology 3:564-571 (1993).

### Polynucleotide Delivery System

A polynucleotide delivery system is any system capable of introducing a polynucleotide, particularly an antigen-specific polynucleotide into a target cell. Polynucleotide delivery systems include both viral and non-viral delivery systems. One of skill in the art will be able to determine the type of polynucleotide delivery system that can be used to effectively deliver a particular antigen-specific polynucleotide into a target cell.

When the antigen-specific polypeptide is a single polypeptide chain, the antigen-specific polynucleotide encoding it is preferably introduced into the target cell in a single polynucleotide delivery system. However, when the antigen-specific polypeptide is a multimeric receptor, for example a dimeric receptor, antigen-specific polynucleotides encoding each of the subunits can be introduced into the target cell, either as a single polynucleotide in a single polynucleotide delivery system, or as separate polynucleotides in one or more polynucleotide delivery systems.

For example, when an antigen-specific polynucleotide encoding a TCR α subunit is to be delivered, it is advantageous to also introduce an antigen-specific polynucleotide encoding a TCR β subunit. If the polynucleotide delivery system has sufficient capacity, the α and β subunits can be introduced together, for example as a single antigen-specific polynucleotide. Thus, in one embodiment the polynucleotide delivery system comprises a polynucleotide encoding a TCR α subunit and a polynucleotide encoding a TCR β subunit. Alternatively, polynucleotides encoding the α and β subunits can be introduced separately into the target cell, each in an appropriate polynucleotide delivery system, for example each as a separate retroviral particle.

The polynucleotide system may comprise one or more polynucleotides in addition to the antigen specific polynucleotides. For example, the polynucleotide delivery system may comprise a polynucleotide that encodes a marker, such as green fluorescent protein (GFP), that can be used to determine if cells have been successfully transfected. The polynucleotide delivery system may also comprise a polynucleotide that encodes a polypeptide that may be used as a "switch" to disable or destroy cells transfected with the antigen specific polynucleotide in a heterogeneous population, for example for safety reasons. In one such embodiment, the gene of interest is a thymidine kinase gene (TK) the expression of which renders a target cell susceptible to the action of the drug gancyclovir.

The polynucleotide deliver system may comprise one or more vectors. The vectors in turn comprise the antigen-specific polynucleotide sequences and/or their complements, optionally associated with one or more regulatory elements that direct the expression of the coding sequences. Eukaryotic cell expression vectors are well known in the art and are available from a number of commercial sources. The choice of vector and/or expression control sequences to which the antigen-specific polynucleotide sequence is operably linked depends directly, as is well known in the art, on the functional properties desired, *e.g*., protein expression, and the target cell to be transformed. A preferred vector contemplated by the present invention is capable of directing the insertion of the antigen-specific polynucleotide into the host chromosome and the expression of the antigen-specific polypeptide encoded by the antigen-specific polynucleotide.

Expression control elements that may be used for regulating the expression of an operably linked antigen-specific polypeptide encoding sequence are known in the art and include, but are not limited to, inducible promoters, constitutive promoters, secretion signals, enhancers and other regulatory elements.

A vector comprising an antigen-specific polynucleotide may include a prokaryotic replicon, *i.e*., a DNA sequence having the ability to direct autonomous replication and maintenance of the recombinant DNA molecule extrachromosomally in a prokaryotic host cell, such as a bacterial host cell, transformed therewith. Such replicons are well known in the art. In addition, vectors that include a prokaryotic replicon may also include a gene whose expression confers a detectable marker such as a drug resistance. Typical bacterial drug resistance genes are those that confer resistance to ampicillin or tetracycline.

The vectors used in the polynucleotide delivery system may include a gene for a selectable marker that is effective in a eukaryotic cell, such as a drug resistance selection marker. This gene encodes a factor necessary for the survival or growth of transformed host cells grown in a selective culture medium. Host cells not transformed with the vector containing the selection gene will not survive in the culture medium. Typical selection genes encode proteins that confer resistance to antibiotics or other toxins, *e.g*., ampicillin, neomycin, methotrexate, or tetracycline, complement auxotrophic deficiencies, or supply critical nutrients withheld from the media. The selectable marker can optionally be present on a separate plasmid and introduced by co-transfection.

Vectors used in the polynucleotide delivery system will usually contain a promoter that is recognized by the target cell and that is operably linked to the antigen-specific polynucleotide. A promoter is an expression control element formed by a DNA sequence that permits binding of RNA polymerase and transcription to occur. Promoters are untranslated sequences that are located upstream (5') to the start codon of a structural gene (generally within about 100 to 1000 bp) and control the transcription and translation of the antigen-specific polynucleotide sequence to which they are operably linked. Promoters may be inducible or constitutive. Inducible promoters initiate increased levels of transcription from DNA under their control in response to some change in culture conditions, such as a change in temperature.

One of skill in the art will be able to select an appropriate promoter based on the specific circumstances. Many different promoters are well known in the art, as are methods for operably linking the promoter to the antigen-specific polynucleotide. Both native promoter sequences and many heterologous promoters may be used to direct expression of the antigen-specific polypeptide. However, heterologous promoters are preferred, as they generally permit greater transcription and higher yields of the desired protein as compared to the native promoter.

The promoter may be obtained, for example, from the genomes of viruses such as polyoma virus, fowlpox virus, adenovirus, bovine papilloma virus, avian sarcoma virus, cytomegalovirus, a retrovirus, hepatitis-B virus and Simian Virus 40 (SV40). The promoter may also be, for example, a heterologous mammalian promoter, *e.g*., the actin promoter or an immunoglobulin promoter, a heat-shock promoter, or the promoter normally associated with the native sequence, provided such promoters are compatible with the target cell.

Transcription may be increased by inserting an enhancer sequence into the vector. Enhancers are cis-acting elements of DNA, usually about 10 to 300 bp in length, that act on a promoter to increase its transcription. Many enhancer sequences are now known from mammalian genes (globin, elastase, albumin, d-fetoprotein, and insulin). Preferably an enhancer from a eukaryotic cell virus will be used. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the antigen-specific polynucleotide sequence, but is preferably located at a site 5' from the promoter.

Expression vectors used in target cells will also contain sequences necessary for the termination of transcription and for stabilizing the mRNA. These sequences are often found in the 5' and, occasionally 3', untranslated regions of eukaryotic or viral DNAs or cDNAs and are well known in the art.

Plasmid vectors containing one or more of the components described above are readily constructed using standard techniques well known in the art.

For analysis to confirm correct sequences in plasmids constructed, the plasmid may be replicated in *E*. *coli,* purified, and analyzed by restriction endonuclease digestion, and/or sequenced by conventional methods.

Vectors that provide for transient expression in mammalian cells of an antigen-specific polynucleotide may also be used. Transient expression involves the use of an expression vector that is able to replicate efficiently in a host cell, such that the host cell accumulates many copies of the expression vector and, in turn, synthesizes high levels of a the polypeptide encoded by the antigen-specific polynucleotide in the expression vector. Sambrook *et al., supra,* pp. 16.17 - 16.22.

Other vectors and methods suitable for adaptation to the expression of antigen-specific polypeptides are well known in the art and are readily adapted to the specific circumstances.

Using the teachings provided herein, one of skill in the art will recognize that the efficacy of a particular delivery system can be tested by transforming primary bone marrow cells with a vector comprising a gene encoding a reporter protein and measuring the expression using a suitable technique, for example, measuring fluorescence from a green fluorescent protein conjugate. Suitable reporter genes are well known in the art.

Transformation of appropriate cells with vectors of the present invention is accomplished by well-known methods, and the method to be used is not limited in any way. A number of non-viral delivery systems are known in the art, including for example, electroporation, lipid-based delivery systems including liposomes, delivery of "naked" DNA, and delivery using polycyclodextrin compounds, such as those described in Schatzlein AG. 2001. Non-Viral Vectors in Cancer Gene Therapy: Principles and Progresses. Anticancer Drugs*.* Cationic lipid or salt treatment methods are typically employed, see, for example, Graham et al. Virol. 52:456, (1973); Wigler et al. Proc. Natl. Acad. Sci. USA 76:1373-76, (1979). The calcium phosphate precipitation method is preferred. However, other methods for introducing the vector into cells may also be used, including nuclear microinjection and bacterial protoplast fusion.

The polynucleotide delivery system is viral. The polynucleotide delivery system comprises a lentiviral vector. belong to this category. Other examples include lentivirus vectors derived from HIV-2, feline immunodeficiency virus (FIV), equine infectious anemia virus, simian immunodeficiency virus (SIV) and maedi/visna virus.

Generation of the viral vector can be accomplished using any suitable genetic engineering techniques well known in the art, including, without limitation, the standard techniques of restriction endonuclease digestion, ligation,transformation, plasmid purification, and DNA sequencing, for example as described in Sambrook et al. (Molecular Cloning: A Laboratory Manual. Cold Spring Harbor Laboratory Press, N.Y. (1989)), Boffin et al. (Retroviruses. Cold Spring Harbor Laboratory Press, N.Y. (1997)) and "RNA Viruses: A Practical Approach" (Alan J. Cann, Ed., Oxford University Press, (2000)).

The viral vector may incorporate sequences from the genome of any known organism. The sequences may be incorporated in their native form or may be modified in any way. For example, the sequences may comprise insertions, deletions or substitutions. In a preferred embodiment the viral vector comprises an intact retroviral 5' LTR and a self-inactivating 3' LTR.

Any method known in the art may be used to produce infectious retroviral particles whose genome comprises an RNA copy of the viral vector. To this end, the viral vector is preferably introduced into a packaging cell line that packages viral genomic RNA based on the viral vector into viral particles with a desired target cell specificity. The packaging cell line provides the viral proteins that are required in *trans* for the packaging of the viral genomic RNA into viral particles. The packaging cell line may be any cell line that is capable of expressing retroviral proteins. Preferred packaging cell lines include 293 (ATCC CCL X), HeLa (ATCC CCL 2), D17 (ATCC CCL 183), MDCK (ATCC CCL 34), BHK (ATCC CCL-10) and Cf2Th (ATCC CRL 1430).

The packaging cell line may stably express the necessary viral proteins. Such a packaging cell line is described, for example, in U.S. Patent No. 6,218,181. Alternatively a packaging cell line may be transiently transfected with plasmids comprising nucleic acid that encodes the necessary viral proteins.

Viral particles are collected and allowed to infect the target cell. Target cell specificity may be improved by pseudotyping the virus. Methods for pseudotyping are well known in the art.

The recombinant retrovirus used to deliver the antigen-specific polypeptide is a modified lentivirus. As lentiviruses are able to infect both dividing and non-dividing cells, it is not necessary to stimulate the target cells to divide.

Recombinant virus produced from the viral vector may be delivered to the target cells in any way that allows the virus to infect the cells. Preferably the virus is allowed to contact the cell membrane, such as by incubating the cells in medium that comprises the virus.

### Target Cells

The target cells are hematopoietic stem cells, primary bone marrow cells, or hematopoietic precursor cells.

Target cells can be derived from any mammalian organism including without limitation, pigs, cows, horses, sheep, goats, rats, mice, rabbits, dogs, cats and guinea pigs. Target cells may be obtained by any method known in the art.

Target cells may be contacted with the polynucleotide delivery system *in vitro.* Preferably, target cells are maintained in culture and are contacted with the polynucleotide delivery system *in vitro.* Methods for culturing cells are well known in the art.

Depending on the polynucleotide delivery system that is to be used, target cell division may be required for transformation. Target cells can be stimulated to divide in vitro by any method known in the art. For example, hematopoietic stem cells can be cultured in the presence of one or more growth factors, such as IL-3, IL-6 and/or stem cell factor (SCF).

### Transgenic Animals

Transgenic animals comprising cells that express a particular antigen-specific polypeptide are also mentioned. An antigen-specific polynucleotide encoding the antigen-specific polypeptide of interest may be integrated either at a locus of a genome where that particular nucleic acid sequence is not otherwise normally found or at the normal locus for the transgene. The transgene may comprise nucleic acid sequences derived from the genome of the same species or of a different species than the species of the target animal.

The antigen-specific polypeptide may be foreign to the species of animal to which the recipient belongs, foreign only to the particular individual recipient, or may comprise genetic information already possessed by the recipient. In the last case, the altered or introduced gene may be expressed differently than the native gene.

While mice and rats remain the animals of choice for most transgenic experimentation, in some instances it is preferable or even necessary to use alternative animal species. Transgenic procedures have been successfully utilized in a variety of non-murine mammals, including sheep, goats, pigs, dogs, cats, monkeys, chimpanzees, hamsters, rabbits, cows and guinea pigs (*see, e.g.,* Kim et al. Mol. Reprod. Dev. 46(4): 515-526 (1997); Houdebine Reprod. Nutr. Dev. 35(6):609-617 (1995); Petters Reprod. Fertil. Dev. 6(5):643-645 (1994); Schnieke et al. Science 278(5346):2130-2133 (1997); and Amoah J. Animal Science 75(2):578-585 (1997)).

Transgenic animals can be produced by a variety of different methods including transfection, electroporation, microinjection, gene targeting in embryonic stem cells and recombinant viral and retroviral infection (*see. e.g*., U.S. Patent No. 4,736,866; US. Patent No. 5,602,307; Mullins et al. Hypertension 22(4):630-633 (1993); Brenin et al. Surg. Oncol. 6(2)99-110 (1997); Tuan (ed.), Recombinant Gene Expression Protocols, Methods in Molecular Biology No. 62, Humana Press (1997)). Detailed procedures for producing transgenic animals are readily available to one skilled in the art, including the disclosures in U.S. Patent No. 5,489,743, U.S. Patent No. 5,602,307 and Lois et al. Science 295(5556):868-872 (2002)).

A transgenic mammal may be produced comprising cells that express a desired antigen-specific polypeptide. The transgenic mammal preferably comprises lymphocytes that express a desired antigen-specific polypeptide, such as a T cell receptor. The mammal may be produced in such a way that substantially all of the lymphocytes express the desired antigen-specific polypeptide. Thus, the transgenic mammal might be produced by a method comprising contacting a non-human embryonic stem cell with a polynucleotide delivery system that comprises an antigen-specific polynucleotide encoding the desired antigen-specific polypeptide. Preferably the polynucleotide delivery system comprises a retroviral vector, more preferably a lentiviral vector.

Alternatively, the transgenic mammal may be produced in such a way that only a sub-population of lymphocytes expresses the desired antigen-specific polypeptide, for example a T cell receptor. Preferably this sub-population of cells has a unique antigen specificity, and does not express any other antigen-specific polypeptides that are capable of inducing an immune response. In particular, the lymphocytes preferably do not express any other T cell receptors. Such mammals might be produced by contacting hematopoietic stem cells with a polynucleotide delivery system comprising an antigen-specific polynucleotide encoding the desired antigen-specific polypeptide. The hematopoietic stem cells are then transferred into a mammal where they mature into lymphocytes with a unique antigen specificity.

### Therapy

The methods of the present invention can be used to prevent or treat a disease or disorder for which an associated antigen can be identified. Diseases or disorders that are amenable to treatment or prevention by the methods of the present invention include, without limitation, cancers, autoimmune diseases, and infections, including viral, bacterial, fungal and parasitic infections.

A mammal that is already suffering from a disease or disorder may be treated. An antigen that is associated with the disease or disorder is identified. The antigen may be previously known to be associated with the disease or disorder, or may be identified by any method known in the art. An antigen-specific polypeptide that recognizes the antigen is then identified. If an antigen-specific polypeptide for the identified antigen is not already known, it may be identified by any method known in the art, as discussed above. Preferably the antigen-specific polypeptide is a T cell receptor.

Target cells are contacted with a polynucleotide delivery system comprising an antigen-specific polynucleotide that encodes the desired antigen-specific polypeptide. Preferably the antigen-specific polynucleotide is a cDNA that encodes the antigen-specific polypeptide. The polynucleotide delivery system preferably comprises a modified lentivirus that is able to infect non-dividing cells, thus avoiding the need for in vitro propagation of the target cells.

The target cells preferably comprise hematopoietic stem cells, more preferably bone marrow stem cells. The target cells are preferably obtained from the mammal to be treated. Methods for obtaining bone marrow stem cells are well known in the art.

Following transfection of the target cells with the antigen-specific polynucleotide, the target cells are reconstituted in the mammal according to any method known in the art.

Also a disease or disorder might be prevented from developing in a mammal. An antigen is identified that is associated with the disease or disorder that is expected to develop. For example, if the disease or disorder is an infection, an antigen is identified that is associated with the infectious agent. Antigens for many diseases and disorders are well known in the art.

A mammal has been or is expected to be exposed to an infectious agent, such as an infectious bacteria or virus, for example HIV. An antigen present on the infectious agent is identified. A polynucleotide that encodes an antigen-specific polypeptide, preferably a T cell receptor that is specific for that antigen, is cloned. Hematopoietic stem cells, preferably bone marrow stem cells, are contacted with a modified retrovirus that comprises the antigen-specific polynucleotide. Preferably the stem cells are obtained from the individual that is expected to be exposed to the infectious agent. Alternatively, they are obtained from another mammal, preferably an immunologically compatible donor. The transfected cells are then transferred into the individual where they develop into mature T cells that are capable of generating an immune response when presented with the antigen from the infectious agent.

Also mentioned are methods used to treat a patient suffering from cancer. An antigen associated with the cancer is identified and an antigen-specific polypeptide that recognizes the antigen is obtained. Preferably the antigen-specific polypeptide is a T cell receptor. An antigen-specific polynucleotide that encodes the antigen-specific polypeptide is cloned. Target cells, preferably hematopoietic stem cells, more preferably primary bone marrow cells, are obtained and contacted with a polynucleotide delivery system that comprises the antigen-specific polynucleotide. The target cells are preferably obtained from the patient, but may be obtained from another source, such as an immunologically compatible donor. The polynucleotide delivery system is preferably a modified retrovirus, more preferably a modified lentivirus. The target cells are then transferred back to the patient, where they develop into cells that are capable of generating an immune response when contacted with the identified antigen.

Also mentioned are methods used for adoptive immunotherapy in a patient. An antigen against which an immune response is desired is identified. A T cell receptor that is specific for the antigen is then identified and a polynucleotide encoding the T cell receptor is obtained. Hematopoietic stem cells, preferably primary bone marrow cells are obtained from the patient and contacted with a polynucleotide delivery system comprising the polynucleotide that encodes the T cell receptor. The target cells are then transferred back into the patient.

After sufficient time to allow the target cells to develop into mature T cells, T lymphocytes are harvested from the patient. This may be done by any method known in the art. Preferably, lymphocytes are isolated from a heterogeneous population of cells obtained from peripheral blood. They may be isolated, for example, by gradient centrifugation, fluorescence activated cell sorting (FACS), panning on monoclonal antibody coated plates or magnetic separation techniques. Antigen specific clones are then isolated by stimulating cells, for example with antigen presenting cells or anti-CD3 monoclonal antibody, and subsequent cloning by limited dilution or other technique known in the art. Clones that are specific for the antigen of interest are identified, expanded and transferred into the patient, such as by infusion into the peripheral blood.

The therapeutic efficacy of an immune response directed against a particular antigen may be assessed in an animal model of a disease state. The immune response might be directed against a previously identified antigen that is known to be associated with the disease state. Alternatively, a previously unknown antigen can be identified. An immune response is provided by generating lymphocytes with a unique specificity for the desired antigen.

For example, the effectiveness of developing an immune response against a known tumor-associated antigen can be tested in a mouse tumor model. In one embodiments hematopoietic stem cells are harvested from a mouse and contacted with a polynucleotide delivery system that comprises a polynucleotide that encodes a T cell receptor that is specific for the tumor associated antigen. The stem cells are then reconstituted in a mouse that has developed or will develop a tumor, where they develop into mature lymphocytes with a unique specificity for the tumor associated antigen. The progression of the tumor in the mouse can be evaluated.

The effectiveness of a specific immune response in preventing the development of a disease or disorder can be determined. A transgenic animal is produced that comprises immune cells that express a desired antigen-specific polypeptide. Isolated antigen is then provided to the transgenic animal, leading to the development of an immune response. The effectiveness of the immune response in preventing the development of the disease or disorder with which the antigen is associated is then measured.

### EXAMPLES

### Experimental Methods

The following experimental methods were used for Examples 1 and 2 described below.

### Mice

C57BL/6 mice were purchased from Charles River, RAG1 and IL-2 knockout mice from Jackson Laboratories. Double IL-2/RAG1 knockout mice were generated by breeding IL-2 knockout mice with RAG1 mice. All mice were housed in Caltech animal facility.

### MIG-TCR retroviruses construction

The MIG retroviral expression vector was created by Dr. Luk Van Parijs (Van Parijs L. et. al, 1999, Immunity, Vol. 11, 281-288). OTII TCRá cDNA and OTII TCRâ cDNA (a gift from Drs Francis Carbone and William Heath, Melbourne, Australia) were cloned separately into the MIG vector using the unique EcoRI restriction site. Retroviruses were generated by culturing 293.T cells in a 6 cm dish till 70-80% confluence and transfecting with the following plasmids using an established calcium phosphate precipitation technique: retroviral plasmid DNA - MIG/OTII á or MIG/TCR â (10µg) and packaging plasmid - pCLEco, (4µg). The DNAs were mixed with 100ul 1.25MCaCl₂, to which we added ddH₂O to 0.5ml, and then 0.5ml 2xBBS (20 ml 0.5 M BES, 22.4 ml 2.5 M NaCl, 600 i 1 0.5 M NaHPO₄ and 157 ml H₂O, pH 6.96) dropwise while bubbling. This mixture was placed on the 293.T cells for 8 hrs, after which the cells were cultured in growth medium. Retrovirus-containing 293.T cell supernatant was collected 48 hr and 72 hr after transfection and used for infection of bone marrow stem cells.

### THZ Hybridoma cell line establishment and infection with retroviruses

Activated mouse CD4+ T cells were fused with the BWZ hybridoma line, which contains a reporter gene (LacZ) that is expressed under the control of the nuclear factor of activated T cells (NFAT) element of the human interleukin-2 promoter (Sanderson S. et. al, 1994, Int. Immunol, 6:369-76), to generate T-cell hybridomas by standard methodology. The hybrids were cloned by limiting dilution. One specific clone was observed to lose TCR expression, while still maintaining CD3 and CD4 expression. This clone was sorted by flow cytometry three times to stabilize the TCR-CD3+CD4+ phenotype. The resulting T cell hybridoma line, THZ, contains endogenous CD3 and CD4, but does not express an endogenous TCR, so it can be used to express sMHC class II-restricted TCRs on its surface. The function of the TCRs expressed was analyzed by lacZ assay.

THZ cells were cultured at 2x10⁶ cells/ml in RPMI Medium 1640 containing 10% FCS. The cells were then spin-infected with a mixture of MIG/OTIIα and MIG/OTIIβ retroviruses in the presence of 10µg/ml polybrene, for 1 hr 30 mins at 2,500 rpm, 30°C. After spin infections, the retroviral supernatant was removed and replaced with growth media. 72hrs later, infected cells were stimulated with residues 323-339 of chicken ovalbumin (OVAp) in the presence of B6 spleen cells as antigen presenting cells (APC) overnight. The next day, OTII TCR response was analyzed by bulk LacZ assay (see below).

### Bulk LacZ Assay

Individual cultures of THZ cells in round-bottom 96-well plates were washed once with 100 µl PUBS then lysed and exposed to the colorogenic β-galactosidase substrate Chlorophenol red β-galactoside (0.15 mM, CPRG, Calbiochem, La Jolla, CA) in the presence of 100 µl Z buffer (100 mM 2-mercaptoethanol, 9 mM MgCl₂, 0.125% NP-40 in PBS, stored at room temperature) and incubated at 37°C overnight. The development of the colored lacZ product was assayed using a plate reader with a 570 nm filter, and a 630 nm filter for reference.

### Bone marrow (BM) stem cell isolation, infection and transfer

RAG1 ko mice, in a wild type or IL-2 knockout background, were treated with 5-FU (5-flurouracil) by intraperitoneal injection of 250 µg 5-FU/gram mouse body weight in PBS. Bone marrow (BM) cells were harvested 5 days later from the tibia and femur of the mice and cultured for 5 days at a density of 2x10⁶ cells/ml with 20 ng/ml rmIL-3, 50 ng/ml rmIL-6, and 50 ng/ml rmSCF (all from Biosource, Camarillo, CA) in DMEM containing 10% FCS. After 48 and 72 hr, the BM cells were spin-infected with mixture of MIG/OTII α and MIG/TCR β retroviruses and 8µg/ml polybrene, for 1 hr 30 mins at 2,500 rpm, at 30°C. After spin infections, the retroviral supernatant was removed and replaced with growth media containing cytokines. Recipient mice of the desired genetic background (RAG mice in wt or IL-2 ko background) received a total 480 rads whole body radiation and then received 1-2x10⁶ infected BM cells by tail vein injection. BM recipient mice were maintained in a sterile environment and were maintained on the mixed antibiotic TMS (Sulfamethoxazole and Trimethoprim oral suspension) (Hi-Tech Pharmacal Co., Amityville, NY) for 11 weeks until analysis.

### BM transferred mice Immunization

Ten weeks after receiving bone marrow, individual mice were immunized by intraperitoneal injection of 200 µg OVAp in 200 µl PBS then left for 6 days till analysis.

### In vitro T cell stimulation and proliferation assay

Spleen cells were harvested and cultured at 2x10⁵ cells/well in flat-bottom 96-well plates with 2x10⁵ cells/well B6 spleen cells as antigen presenting cells (APC) in standard T cell medium containing OVAp at 0, 0.01, 0.1, 1, or 10 µg/ml. Three days later, culture supernatant were collected and used for IL-2 and INF-ã ELISA. ³H thymidine was added to the wells at a final concentration of 0.01 mCi/ml. These cells were incubated for another 24 hours, sealed and kept at 20°C until ³H counting. Data was collected with a Wallac ³H counter.

### IL-2 and INF- ã ELISA

96-well ELISA plates were coated with purified anti-mIL-2 or anti-INF ã antibody (Pharmingen, San Diego, CA) diluted in carbonate buffer (0.1 M sodium bicarbonate, 0.1 M sodium carbonate, pH 9.4, stored at RT) to 1 ì g/ml, by adding 50 µl/well and incubating for 2 hrs at 37°C or 4 hr at room temperature (RT) or overnight (O/N) at 4°C. The plates were then washed twice with PBS, blocked by adding 100 µl/well of dilution buffer BBS/2% BSA/0.002% azide, incubated for 30 min at 37°C or 1 hr at RT or O/N at 4°C. Then after being washed 4 times with PBS, sample supernatants were added to the plates at final volume of 50 µl/well, incubated for 3 hrs at 37°C or 6 hrs at RT or O/N at 4°C. The plates were then washed 4 times followed by addition of 50 µl/well of the detecting biotinylated antibody (Pharmingen, San Diego, CA) diluted in the dilution buffer BBS/2% BSA/0.002%azide and incubated for 45 min at RT. Next the plates were washed 6 times with PBS, 50 µl/well of the Avidin-Alkaline Phosphotase (Pharmingen, San Diego, CA) diluted 1:400 in the dilution buffer BBS/2% BSA/0.002% azide was added and they were incubated for 30 min at RT. Then the plates were washed 6 times with PBS. Developing solution Sigma 104 Phosphatase Substrate (Sigma, ST. Louis, MO) was made at 1 mg/ml in DEA buffer (24.5 mg MgCl₂.6H₂O, 48 ml diethanolamine in 400 ml dH₂O, pH to 9.8 with HCl, made up to 500ml and stored in a foil wrapped bottle at RT) right before use and then added at 50 µl/well (light sensitive therefore kept foil wrapped). Data was collected with a plate reader at 405 nm.

### Example 1

### In vitro demonstration of functional expression of antigen-specific TCRs using retroviral vector

This example demonstrates the successful expression of a functional TCR in a hybridoma cell line. The bicistronic MIG retroviral expression vector was created by placing GFP downstream of the pCITE1 IRES (Novagen) and cloning it into MSCV 2.2 vector (Van Parijs et al. 1999, Immunity, Vol. 11, 281-288). This retroviral vector (shown in Figure 1A) expresses both GFP, to mark infected cells, and a heterologous gene of interest. OTII T Cell Receptor (TCR) α or β chain cDNAs were cloned into this vector. The OTII TCR is a well-defined TCR derived from a CD4+ class II-restricted T cell clone that responds to a known antigen, residues 323-339 of chicken ovalbumin (OVAp). The OTII TCR was used as a model system in our experiments.

OTII TCRá /MIG and OTII TCRâ/MIG retroviruses were used to double-infect the THZ hybridoma cell line. This cell line has expresses endogenous CD3, so it can express TCRs on its surface. The cell line also contains a reporter gene (LacZ) that is expressed under the control of the nuclear factor of activated T cells (NFAT) element of the human interleukin-2 promoter, and can be used to assay TCR signaling. The left panel of Figure 1B shows that infected THZ cells (identified by expression of the GFP marker gene) expressed OTII TCR on surface. The right panel of Figure 1B shows that these cells signaled through the TCR in response to OVAp, proving that functional expression of OTII TCR was obtained using MIG retroviruses.

It was also demonstrated that a functional TCR could be expressed in primary T cells using retroviruses. Purified CD4+ T cells from wild type C57BL/6 mice were activated with an antibody to CD3å and infected with MIG OTIIα and MIG OTIIβ viruses. The infected T cells (marked by GFP fluorescence) expressed the β chain of the OTII TCR at the cell surface and proliferated when cultured with OVAp presented by APCs (Figure 1C).

### Example 2

### Generation of functional antigen-specific T cells in mice of defined genetic background

Figure 2 shows schematically the methods of the invention applied to the generation of a transgenic mouse. Bone marrow cells were obtained from mice of the desired genetic background (in these experiments, wild type or IL-2 knockout RAG1-deficient mice) and infected them with retrovirus expressing the TCR gone, as described above. The infected BM cells were then transferred into a lethally irradiated RAG1 deficient host mouse and allowed to reconstitute functionally normal T cells.

In both wild type (wt) and IL-2 knock-out (IL-2 ko) RAG1-deficient genetic backgrounds, expression of the OTII TCRα and β cDNAs in stem cells by the MIG retrovirus led to the development of phenotypically normal OT.II CD4+ T cells in the thymi of host mice. The cellularity of the thymi derived from mice expressing OTIIá and â chains was greatly increased compared to those from control mice that received bone marrow precursor cells infected with the empty MIG vector.

The upper panels of Figure 3A show the presence of GFP+ cells in the thymus of host mice, indicating that they were derived from retrovirally-transduced RAG1 deficient wild type or IL-2 knockout stem cells. In fact, the majority (>80%) of cells in the thymi of mice receiving OTII-expressing cells were GFP positive. These thymocytes showed the expected distribution of CD4 and CD8 markers for developing class II-restricted T cells. The lower panels of Figure 3B show that the GFP+ cells developed into mature CD4 single positive T cells.

In both wild type and IL-2 knockout RAG-1 deficient genetic backgrounds, expression of the OTII TCRα and β cDNAs in stem cells by the MIG retrovirus led to the accumulation of phenotypically normal OT.II CD4+ T cells in the peripheral lymphoid organs such as lymph nodes and the spleen. The upper panels of Figure 3B show the presence of lymph node cells expressing GFP (GFP+) indicating that they were derived from retrovirally-transduced BM stem cells. From 30 to 60% of the cells in the lymph nodes and spleens of the mice were GFP positive. The lower panels of Figure 3B shows that the GFP+ cells were CD4+ T cells expressing the OTII TCR. More than 80% of these cells were mature CD4+ T cells that expressed the OTII Vβ element, Vβ5. These results demonstrated that retrovirus-mediated expression of TCR cDNAs in bone marrow precursor cells could drive normal T cell development.

Figure 3C illustrates the normal functional responses of OTII TCR transgenic CD4+ T cells obtained from the peripheral lymphoid organs of mice receiving retrovirally-transduced bone marrow stem cells.

OTII TCR transgenic CD4+ T cells in both wt and IL-2 ko genetic backgrounds showed the expected response to antigen. OT.II TCR transgenic CD4+ T cells were obtained from the spleens of BM transfer host mice and were stimulated with increasing concentrations of OVAp *in vitro.* The upper panels of Figure 3C show that OTII TCR. transgenic CD4+ T cells in a wt genetic background responded as expected of normal naive T cells to OVAp; they proliferated and secreted IL-2 when stimulated. The middle and lower panels of Figure 3C show the response of OTII TCR transgenic CD4+ T cells in IL-2 ko genetic background to OVAp. As expected, these cells proliferated poorly in the absence of IL-2 and did not secrete IL-2. Addition of exogenous IL-2 stimulated proliferation in the presence of antigen.

Figure 4A shows the normal cell expansion and expression of activation markers following *in vivo* antigen stimulation of OTII TCR transgenic CD4+ T cells in the peripheral lymphoid organs of mice receiving retrovirally-transduced bone marrow stem cells. Host mice that received retrovirally-transduced wild type or IL-2 knockout bone marrow stem cells show the expected expansion and activation of OTII TCR transgenic CD4+ T cells following immunization with OVAp. In both genetic backgrounds, the OTII TCR transgenic CD4+ T cells expanded and expressed activation markers that mark the transition from naive to effector T cell (CD69, CD62L and CD44). The upper panels of Figure 4A show the expansion and induction of activation markers on OTII transgenic T cells in immunized wild type mice. The bottom panel of Figure 4A shows the same for IL-2 knockout mice.

Figure 4B shows the preferential expansion of GFP^{high} OTII TCR transgenic CD4+ T cells following stimulation with antigen *in vivo.* Following immunization with OVAp a preferential expansion of GFP^{high} OTII TCR transgenic CD4+ T cells was observed. Since the expression of GFP correlates with expression of TCR in this system, this result indicates that the selected T cells expressed higher amounts of the OTII TCRα and TCRβ chains. This result suggests that it is possible to select the optimal cells to respond to an immunological challenge *in vivo* using this gene delivery strategy.

Figure 4C shows normal functional responses of OTII TCR transgenic CD4+ T cells following *in vivo* stimulation with antigen. OTII TCR transgenic CD4+ T cells that were stimulated with antigen *in vivo* acquired effector functions. OT.II TCR transgenic CD4+ T cells in both wt and IL-2 ko genetic backgrounds were obtained from the spleens of immunized mice. These cells were stimulated with OVAp *in vitro.* The upper panels of Figure 4C shows that immunized OTII TCR transgenic CD4+ T cells in wt genetic background performed enhanced proliferation to OVAp and secreted IFNγ. These are characteristics of functional effector T cells. The middle and lower panels of Figure 4C show the response of primed OTII TCR transgenic CD4+ T cells in IL-2 ko genetic background to OVAp, restimulated with (lower) or without (upper) exogenous IL-2. These cells show the expected dependence on IL-2 for proliferation and IFNγ production.

These results demonstrated that retrovirus-mediated expression of TCR cDNAs in bone marrow precursor cells could give rise to functionally mature T cells on different genetic backgrounds that respond normally to antigen exposure in vivo.

### Example 3

### Generation of Wild Type Mice Expressing Antigen-Specific TCRs

The ability to generate wild-type mice expressing antigen-specific TCRs was investigated. Bone marrow cells were obtained from wild-type B6 mice that had been previously treated with 5-fluorouracil as described above. Bone marrow cells were infected with the MIG retrovirus comprising sequences encoding the OTII TCRá and TCRâ subunits, as well as a GFP marker protein. The infected bone marrow cells were then transferred into an irradiated host animal and allowed to reconstitute functionally normal T cells.

As can be seen in Figure 6A, approximately 65% of the cells extracted from the thymi of mice receiving infected BM cells expressed GFP. Figure 6B shows that of the CD4+GFP+ thymocytes, about 21% expressed the OTII Vβ element. Further, the GFP positive thymocytes showed normal distribution of CD4 and CD8 markers (Figure 6C).

In addition, infected BM cells were found to develop into mature CD4+ T cells expressing transgenic TCRs in the peripheral lymph nodes. Figure 7A shows that approximately 44% of the cells in the peripheral lymph nodes were GFP positive. Many of the GFP positive cells were CD4+ T cells expressing OTII TCR Vβ (Figure 7B and 7C), indicating that retrovirus mediated expression of TCR cDNAs in wild type bone marrow precursor cells can result in normal T cell development in a host.

### Example 4

### In vitro demonstration of functional expression of antigen-specific TCRs using lentiviral vector

A tri-cistronic lentiviral vector was constructed based on the lentiviral vector described in (Lois et al., Science 295:868-872 (2002); U.S. Patent Publication No. 2003/0101472). A diagram of the vector is shown in Figure 8. Briefly, cDNAs encoding OTII TCRα and β and GFP were cloned separately into the FUW lentiviral vector. The cDNAs were separated by internal ribosome entry site (IRES) elements (U.S. Patent No. 4,937,190). The vector also comprised an ubiquitin promoter (Ubi) and a woodchuck hepatitits virus response element (WRE; Zufferey et al. J. Virol. 74:3668-3681 (1999); Deglon et al. Hum. Gene Ther. 11:179-190 (2000)), as indicated.

Recombinant lentivirus was generated by co-transfecting 293 cells with the lentiviral vector and packaging vectors VsVg, pRRE and pRSV rev (Yee et al. Methods Cell Biol. 43A:99-112 (1994); Dull et al. J. Virol. 72(11):8463-8471 (1998)). Retrovirus was collected and titred and used for infection of bone marrow stem cells.

The recombinant lentivirus is advantageous because it is able to infect non-dividing cells. As a result, bone marrow cells do not need to be stimulated in vitro and manipulations can be minimized.

Infection of naive T cells with the tri-cistronic recombinant lentivirus was found to mediate expression of functional OTII TCR that is able to respond to antigen challenge. As diagrammed in Figure 9A, spleen cells were obtained from wild-type B6 mice and infected with the recombinant lentivirus. The spleen cells were then stimulated with Ova. The infected spleen cells showed proliferation in response to Ova stimulation. FACS analysis of cells after 3 days stimulation with Ova showed that the majority of the cells were GFP+ and expressed both OTII TCR α and β. The left panel of Figure 9B shows that nearly all cells were GFP positive, indicating that they were successfully infected. The right panel of Figure 9B indicates that greater than 90% of the cells express both OTII TCR α and β. The preferential proliferation and expansion of infected cells means that these cells responded to antigen challenge. Detection of OTII α and β expression on these cells confirmed tri-cistronic recombinant lentivirus mediated functional expression of antigen specific TCR.

### Example 5

### Lentivirus infection of fresh isolated BM mediated stable gene transfer into hematopoietic stem cells

The efficiency and stability of lentiviral mediated gene transfer into freshly isolated hematopoietic stem cells was investigated. Bone marrow cells were obtained from untreated wild-type mice and infected with FUW lentivirus comprising a GFP marker gene. The infected bone marrow cells were then transferred into a wild-type host mouse that had received sub-lethal irradiation (Figure 10), where they were allowed to develop into mature T cells. Cells in the bone marrow, thymus and peripheral lymph nodes were then extracted and analyzed for GFP expression. As shown in Figure 11A, all three compartments comprised a significant number of cells that expressed the GFP transgene. In addition, both B cells and T cells showed expression of the transgene (Figure 12A), indicating that the transgene was integrated into hematopoietic stem cells.

Bone marrow cells from the first host mouse were then transferred into a second host mouse (Figure 10). The bone marrow cells were not manipulated in any way during the transfer. As can be seen in Figure 11B, GFP expression was maintained in the bone marrow, thymus and peripheral lymph nodes in the second host mouse. Further, GFP expression was seen in both B cells and T cells (Figure 12B). These results indicate that the transgene was stably integrated into hematopoietic stem cells and would not be silenced by time.

### References

Barnden, M. J., Allison, J., Heath, W. R., and Carbone, F. R. (1998). Defective TCR expression in transgenic mice constructed using cDNA-based alpha- and beta-chain genes under the control of heterologous regulatory elements. Immunol Cell Biol 76, 34-40.
Berg, L. J., Fazekas de St Groth, B., Ivars, F., Goodnow, C. C., Gilfillan, S., Garchon, H. J., and Davis, M. M. (1988). Expression of T-cell receptor alpha-chain genes in transgenic mice. Mol Cell Biol 8, 5459-69.
Bluthmann, H., Kisielow, P., Uematsu, Y., Malissen, M., Krimpenfort, P., Berns, A., von Boehmer, H., and Steinmetz, M. (1988). T-cell-specific deletion of T-cell receptor transgenes allows functional rearrangement of endogenous alpha- and beta-genes. Nature 334, 156-9.
Clay, T. M., Custer, M. C., Sachs, J., Hwu, P., Rosenberg, S. A., and Nishimura, M. I. (1999). Efficient transfer of a tumor antigen-reactive TCR to human peripheral blood lymphocytes confers anti-tumor reactivity. J Immunol 163, 507-13.
Cooper, L. J., Kalos, M., Lewinsohn, D. A., Riddell, S. R., and Greenberg, P. D. (2000). Transfer of specificity for human immunodeficiency virus type 1 into primary human T lymphocytes by introduction of T-cell receptor genes. J Virol 74, 8207-12.
Deglon et al. Hum. Gene Ther. 11:179-190 (2000)
Dembic, Z., Haas, W., Weiss, S., McCubrey, J., Kiefer, H., von Boehmer, H., and Steinmetz, M. (1986). Transfer of specificity by murine alpha and beta T-cell receptor genes. Nature 320, 232-8.
Dull et al. J. Virol. 72(11):8463-8471 (1998)
Fujio, K., Misaki, Y., Setoguchi, K., Morita, S., Kawahata, K., Kato, I., Nosaka, T., Yamamoto, K., and Kitamura, T. (2000). Functional reconstitution of class II MHC-restricted T cell immunity mediated by retroviral transfer of the alpha beta TCR complex. J Immunol 165, 528-32.
Kessels, H. W., Wolkers, M. C., van den Boom, M. D., van der Valk, M. A., and Schumacher, T. N. (2001). Immunotherapy through TCR gene transfer. Nat Immunol 2, 957-61.
Kouskoff, V., Signorelli, K., Benoist, C., and Mathis, D. (1995). Cassette vectors directing expression of T cell receptor genes in transgenic mice. J Immunol Methods 180, 273-80.
Lois et al., Science 295:868-872 (2002)
Mamalaki, C., Elliott, J., Norton, T., Yannoutsos, N., Townsend, A. R, Chandler, P., Simpson, E., and Kioussis, D. (1993). Positive and negative selection in transgenic mice expressing a T-cell receptor specific for influenza nucleoprotein and endogenous superantigen. Dev Immunol 3, 159-74.
Moss, P. A. (2001). Redirecting T cell specificity by TCR gene transfer. Nat Immunol 2, 900-1.
Pircher, H., Burki, K., Lang, R., Hengartner, H., and Zinkernagel, R. M. (1989). Tolerance induction in double specific T-cell receptor transgenic mice varies with antigen. Nature 342, 559-61.
Stanislawski, T., Voss, R. H., Lotz, C., Sadovnikova, E., Willemsen, R. A., Kuball, J., Ruppert, T., Bolhuis, R. L., Melief, C. J., Huber, C., Stauss, H. J., and Theobald, M. (2001). Circumventing tolerance to a human MDM2-derived tumor antigen by TCR gene transfer. Nat Immunol 2, 962-70.
Uematsu, Y., Ryser, S., Dembic, Z., Borgulya, P., Krimpenfort, P., Berns, A., von Boehmer, H., and Steinmetz, M. (1988). In transgenic mice the introduced functional T cell receptor beta gene prevents expression of endogenous beta genes. Cell 52, 831-41.
Yee et al. Methods Cell Biol. 43A:99-112 (1994)
Zufferey et al. J. Virol. 74:3668-3681 (1999)

## Claims

1. *In vitro* method of generating a lymphocyte with a unique antigen specificity in a mammal comprising:
contacting a mammalian stem cell *in vitro* with a modified lentivirus polynucleotide delivery system comprising an antigen-specific polynucleotide, encoding a T cell receptor α subunit and a T cell receptor β subunit under the control of one promoter, wherein the mammalian stem cell is a hematopoietic stem cell, a primary bone marrow cell or a hematopoietic precursor cell.

2. Lymphocyte with a unique antigen specificity obtainable by the method according to claim 1.

3. Use of a lymphocyte with a unique antigen specificity according to claim 2 in the preparation of a medicament for stimulating an immune response to the antigen in a mammal.

4. Lymphocyte with a unique antigen specificity obtainable by the method according to claim 1, the method comprising the following additional steps: cloning a T cell that expresses the T cell receptor on its surface obtained from a patient and expanding the T cell *in vitro.*

5. Use of a lymphocyte with a unique antigen specificity according to claim 2 or 4 in the preparation of a medicament for the treatment of cancer, wherein the antigen is associated with cancer.

6. Use of a lymphocyte with a unique antigen specificity according to claim 2 in the preparation of a medicament for preventing infection in a mammal that has been or is expected to be exposed to an infectious agent, wherein the antigen is associated with the infectious agent.

7. Use according to claim 6, wherein the infectious agent is HIV.

## Patentansprüche

1. *In vitro* Verfahren zum Erzeugen eines Lymphozyten mit einer einzigartigen AntigenSpezifität in einem Säugetier, umfassend:
Inkontaktbringen einer Stammzelle eines Säugetiers *in vitro* mit einem modifizierten Lentivirus-Polynukleotid-Zuführungssystem, das ein antigen-spezifisches Polynukleotid umfasst, das unter der Kontrolle eines Promotors eine T-Zellrezeptor α-Untereinheit und eine T-Zellrezeptor β-Untereinheit kodiert, wobei die Stammzelle des Säugetiers eine hämatopoetische Stammzelle, eine primäre Knochenmarkszelle oder eine hämatopoetische Vorläuferzelle ist.

2. Lymphozyt mit einer einzigartigen Antigenspezifität erhältlich durch das Verfahren nach Anspruch 1.

3. Verwendung eines Lymphozyten mit einer einzigartigen Antigenspezifität nach Anspruch 2 bei der Herstellung eines Medikaments zum Stimulieren einer Immunantwort gegenüber dem Antigen in einem Säugetier.

4. Lymphozyt mit einer einzeigartigen Antigenspezifität erhältlich durch das Verfahren nach Anspruch 1, wobei das Verfahren die folgenden weiteren Schritte umfasst: Klonieren einer den T-Zellrezeptor auf der Oberfläche exprimierenden T-Zelle, die von einem Patienten erhalten wurde, und Expandieren der T-Zelle *in vitro.*

5. Verwendung eines Lymphozyten mit einer einzigartigen Antigenspezifität nach Anspruch 2 oder 4 bei der Herstellung eines Medikaments zur Behandlung von Krebs, wobei das Antigen mit Krebs assoziiert ist.

6. Verwendung eines Lymphozyten mit einer einzigartigen Antigenspezifität nach Anspruch 2 bei der Herstellung eines Medikaments zum Verhindern einer Infektion in einem Säugetier, das einem infektiösen Agens ausgesetzt ist oder von dem erwartet wird, diesem ausgesetzt zu sein, wobei das Antigen mit dem infektiösen Agens assoziiert ist.

7. Verwendung nach Anspruch 6, wobei das infektiöse Agens HIV ist.

## Revendications

1. Procédé de génération *in vitro* d'un lymphocyte comportant une spécificité antigénique unique chez un mammifère comprenant :
contacter *in vitro* une cellule souche de mammifère avec un système de libération de polynucléotide de lentivirus modifié comprenant un polynucléotide spécifique d'antigène, codant une sous-unité α de récepteur de cellule T et une sous-unité β de récepteur de cellule T sous le contrôle d'un promoteur, dans lequel la cellule souche de mammifère est une cellule souche hématopoïétique, une cellule primaire de moelle osseuse ou une cellule précurseur hématopoïétique.

2. Lymphocyte comportant une spécificité antigénique unique susceptible d'être obtenu par le procédé selon la revendication 1.

3. Utilisation d'un lymphocyte comportant une spécificité antigénique unique selon la revendication 2 dans la préparation d'un médicament pour stimuler chez un mammifère une réponse immunitaire à un antigène.

4. Lymphocyte comportant une spécificité antigénique unique susceptible d'être obtenu par le procédé selon la revendication 1, le procédé comprenant l'étape additionnelle suivante : cloner une cellule T obtenue partir d'un patient qui exprime le récepteur de cellule T à sa surface et multiplier la cellule T in vitro.

5. Utilisation d'un lymphocyte comportant une spécificité antigénique unique selon la revendication 2 ou 4 dans la préparation d'un médicament pour le traitement du cancer dans laquelle l'antigène est associé au cancer.

6. Utilisation d'un lymphocyte comportant une spécificité antigénique unique selon la revendication 2 dans la préparation d'un médicament pour prévenir une infection chez un mammifère qui a été ou qui va être exposé à un agent infectieux dans laquelle l'antigène est associé à l'agent infectieux.

7. Utilisation selon la revendication 6 dans laquelle l'agent infectieux est le VIH.
